(19)

Europäisches
Patentamt

European
Patent Office

Office européen
des brevets

(11)  **EP 3 266 745 A1**

(12)  **EUROPEAN PATENT APPLICATION**
published in accordance with Art. 153(4) EPC

(43) Date of publication:
**10.01.2018 Bulletin 2018/02**

(21) Application number: **16759022.3**

(22) Date of filing: **03.03.2016**

(51) Int Cl.:
*C01B 33/44* [(2006.01)]      *C01B 33/42* [(2006.01)]
*C08K 9/04* [(2006.01)]       *C08L 101/00* [(2006.01)]

(86) International application number:
**PCT/JP2016/056670**

(87) International publication number:
**WO 2016/140330 (09.09.2016 Gazette 2016/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **05.03.2015   JP 2015043961**

(71) Applicants:
• **Hitachi Chemical Company, Ltd.**
 **Chiyoda-ku**
 **Tokyo 100-6606 (JP)**
• **National Institute Of Advanced Industrial Science**
 **Tokyo 100-8921 (JP)**

(72) Inventors:
• **SONG, Shihui**
 **Tokyo 100-6606 (JP)**
• **FUKUSHIMA, Keiji**
 **Tokyo 100-6606 (JP)**
• **TAKEZAWA, Yoshitaka**
 **Tokyo 100-6606 (JP)**
• **HOTTA, Yuji**
 **Nagoya-shi**
 **Aichi 463-8560 (JP)**
• **IMAI , YUSUKE**
 **Nagoya-shi**
 **Aichi 463-8560 (JP)**
• **SHIMAMOTO, Daisuke**
 **Nagoya-shi**
 **Aichi 463-8560 (JP)**
• **TOMINAGA, Yuichi**
 **Nagoya-shi**
 **Aichi 463-8560 (JP)**

(74) Representative: **Hoffmann Eitle**
 **Patent- und Rechtsanwälte PartmbB**
 **Arabellastraße 30**
 **81925 München (DE)**

(54)  **COMPLEX BETWEEN LAMELLAR INORGANIC COMPOUND AND ORGANIC COMPOUND AND METHOD FOR PRODUCING SAME, EXFOLIATED LAMELLAR INORGANIC COMPOUND AND METHOD FOR PRODUCING SAME, INSULATING RESIN COMPOSITION, RESIN SHEET, INSULATOR, RESIN SHEET CURED ARTICLE, AND THERMAL DISSIPATION MEMBER**

(57)      A method of producing a complex of a lamellar inorganic compound and an organic compound includes: heat-treating a particular non-swelling lamellar inorganic compound within a pyrolysis temperature range of the non-swelling lamellar inorganic compound; and intercalating an organic compound into the non-swelling lamellar inorganic compound in a dispersion liquid in which the heat-treated non-swelling lamellar inorganic compound is dispersed in a medium, thereby inserting the organic compound into an interlamellar space of the non-swelling lamellar inorganic compound.

**EP 3 266 745 A1**

**Description**

Technical Field

The present invention relates to a complex of a lamellar inorganic compound and an organic compound and a method of producing thereof, a delaminated lamellar inorganic compound and a method of producing thereof, an insulating resin composition, a resin sheet, an insulator, a resin sheet cured product, and a heat dissipating member.

Background Art

Materials for high voltage apparatuses such as power generators, rotating electrical machines, and electric transmission/substation equipment are equipped with insulating members for blocking between conductive members for passage of the electric current and conductors or between conductors and the ground. For insulating members of such high voltage apparatuses, in view of insulation, chemical stability, mechanical strength, heat resistance, cost, and the like, insulating resin materials using usual epoxy resins as base materials are used.

For the intended use described above, various properties, for example, excellent insulating and voltage resistance properties as electrical properties, high thermal conductance and heat resistance as thermal properties, and high rigidity, high flexibility, and adhesiveness as mechanical properties, and gas barrier properties are needed. In order to ensure such properties, Japanese Patent Application Laid-Open (JP-A) No. 2008-75069 discloses filling an epoxy resin with an inorganic compound such as a silica, alumina, or a smectite-based clay compound.

As an aside, JP-A No. H9-208745 and JP-A No. 2008-7753 disclose attempts to disperse a lamellar inorganic compound such as mica in a thermoplastic resin such as polypropylene or polyamide for compounding, thereby improving properties of the composite material such as insulating, voltage resistance, and heat resistance properties. In these prior art technologies, in a case in which a thermoplastic resin and a lamellar inorganic compound are dispersed, a method of melt kneading the lamellar inorganic compound into the thermoplastic resin is employed.

However, in a method of melt-kneading a lamellar inorganic compound into a thermoplastic resin, heat is added to the thermoplastic resin such as polypropylene, which causes the lamellar inorganic compound to be dispersed. Therefore, the method cannot be applied for a thermosetting resin such as an epoxy resin, which is difficult to melt-knead by heat.

In addition, a lamellar inorganic compound originally contains an alkali metal such as sodium or potassium, that shows interlamellar hydrophilicity. In addition, as it has hydroxyl groups on the crystal surface, it has affinity for polar solvents such as water but it has low affinity for organic solvents and organic substances such as epoxy resins. This causes aggregation of the lamellar inorganic compound and generation of voids accompanied with the aggregation when the lamellar inorganic compound is kneaded with a resin lamellar inorganic compound, which makes it difficult to disperse the lamellar inorganic compound uniformly in the resin, resulting in deterioration of properties such as insulating, voltage resistance, and heat resistance properties. This has been problematic.

Moreover, as it is required for insulating resin materials to have high reliability, filling with a certain amount or more of a lamellar inorganic compound is necessary for the improvement of properties such as more excellent insulating, high thermal conductance, and voltage resistance properties. However, when the filling rate is increased, a resin does not enter a space around the lamellar inorganic compound, which tends to cause void formation. This is problematic because of manufacturing cost increase as well as deterioration of a property of insulating resin material. In order to solve such problem, it is effective to increase an aspect ratio of the lamellar inorganic compound, that is to say, to increase a specific surface area. JP-A No. H9-87096 reports that a composite material of a smectite-based clay compound that is a lamellar inorganic compound having an increased aspect ratio and a resin has improved mechanical characteristics. As in the case of the smectite-based clay compound, it is considered that when mica that is a lamellar inorganic compound has a higher aspect ratio, a material obtained as a result of compounding of mica and a resin has a higher effect of improving properties such as insulating, voltage resistance, heat resistance properties. Therefore, in order to achieve these objects, there is a demand for development of technology of delaminating a lamellar inorganic compound (nano-sheeting technology).

In order to effectively delaminate a lamellar inorganic compound, it is effective to decrease interlamellar binding force. Nano sheets in the lamellar inorganic compound are very strongly bound to each other via covalent binding or the like. Meanwhile, an interlamellar space of a lamellar inorganic compound is formed via relatively weak binding due to van der Waals' force, electrostatic interaction, or the like. The van der Waals' force is represented by dispersion force of the Lennard-Jones potential shown in Equation (1) (the senary member in the formula), which is known to be inversely proportional to the six power of distance r. In addition, electrostatic interaction can be expressed by Equation (2), it is known to be inversely proportional to distance r. As stated above, since it is possible to weaken binding force by expanding interlamellar distance, there is a demand for development of technology of expanding interlamellar distance in order to effectively achieve delamination.

$$U(r) = 4\varepsilon\{(\delta/r)^{12} - (\delta/r)^6\} \quad (1)$$

$$U(r) = -(q_+ q_-)/4\pi\varepsilon_0\varepsilon_r r \quad (2)$$

In the Equation, U (r) denotes potential energy of an arbitrary molecular pair, $\varepsilon$ and $\delta$ denote fitting parameters particular to molecules, q+ and q- denote charge amounts, $\varepsilon_r$ denotes relative permittivity of a medium, $\varepsilon_0$ denotes vacuum permittivity, and r denotes distance.

As stated above, in order to uniformly disperse a lamellar inorganic compound in a resin such as an epoxy resin, it is essential to improve affinity between the lamellar inorganic compound and the resin and develop technology of delaminating the lamellar inorganic compound. Therefore, in order to solve this issue, the inventors conducted intensive studies on a method of intercalating an organic compound into an interlamellar space of a lamellar inorganic compound (intercalation), a complex of a lamellar inorganic compound and an organic compound and a method of producing thereof (i.e., organification treatment), and a delaminated lamellar inorganic compound and a method of producing thereof.

Intercalation is a phenomenon in which atoms, molecules, or the like enter into an interlamellar space of a lamellar inorganic compound. Since the phenomenon does not cause a change in a crystal structure before and after intercalation, it is used, as a pretreatment of delamination of a lamellar inorganic compound or an operation for improving affinity between a resin and a lamellar inorganic compound, for a clay compound such as smectite. A lamellar inorganic compound that has been subjected to organification treatment via intercalation shows affinity for resins such as nylon resins. JP-A No. S63-215775 discloses that a lamellar inorganic compound and an organic compound such as a monomer are kneaded and polymerized, thereby uniformly dispersing the lamellar inorganic compound in a resin. Further, JP-A No. 2004-169030 discloses attempts to conduct dispersion treatment of a lamellar inorganic compound that has been subjected to organification treatment under severe conditions such as ultrasound irradiation so as to obtain a crushed lamellar inorganic compound.

In addition, in order to improve insulating property of a resin composition to suppress, for example, progress in electrical treeing, a technique of dispersing nanometer-size inorganic nanoparticles , that is a lamellar inorganic compound, in a resin composition has been used so far.

For example, JP-A No. 2009-191239 discloses that a lamellar inorganic compound that has been subjected to organification treatment is made to swell with an organic solvent by interlamellarly inserting an organic compound into a lamellar inorganic compound via ion exchange treatment, and then, the lamellar inorganic compound is kneaded with a resin. As a result, interlamellar delamination of the lamellar inorganic compound takes place, thereby allowing each layer of the delaminated lamellar inorganic compound to be uniformly dispersed in the resin. In this regard, JP-A No. 2009-191239 discloses that a resin composition having improved resistance to partial discharging can be obtained.

Further, JP-A No. 2012-158622 discloses a method of producing a resin composition for high voltage apparatuses which is imparted with improved insulating property by having a lamellar inorganic compound to swell with water or a water-based mixed solvent and to have organic functional groups using a silane coupling agent and kneading the lamellar inorganic compound therewith.

JP-A No. 2008-63408 and WO2006/22431 disclose using mica, that is a lamellar inorganic compound, in contrast to clay, that is a lamellar inorganic compound disclosed in JP-ANo. 2009-191239 and JP-ANo. 2012-158622.

Specifically, JP-A No. 2008-63408 discloses that it was found that delamination dispersibility of mica is promoted by melt-kneading, by using a kneader, a resin and an intercalation compound, which is obtained by intercalating an organic modifier into mica, that is a lamellar inorganic compound, at the evaporation temperature of an organic modifier contained in the intercalation compound.

WO2006/22431 discloses an organic-inorganic complex which is obtained by treating a non-swelling mica having a large primary particle diameter in a concentrated solution of a positively charged organic compound, and also discloses a polymer composite material in which the organic-inorganic complex has been favorably dispersed.

SUMMARY OF INVENTION

Technical Problem

The method disclosed in JP-A No. S63-215775 employs a polymerization reaction. Therefore, it is necessary to consider a reaction method depending on the types and amounts of a resin monomer serving as a base and an organic compound used for intercalation, which is problematic due to increase in manufacturing cost. In addition, according to the method disclosed in JP-A No. 2004-169030, ultrasounds cause mica to be not delaminated but crushed, and therefore, a length of mica in the longitudinal direction (a axis) is shortened, which is problematic due to a decrease in an aspect ratio. Further, a swellable inorganic compound in which sodium ions are contained between layers of a clay compound such as smectite swells by incorporating water, an organic solvent, or the like between the layers, which tends to cause intercalation. However, such phenomenon is unlikely to be induced in a non-swelling mica containing potassium ions between its layers, which is problematic because it is difficult to cause intercalation.

Further, for the purpose of improving insulating property of a resin composition, JP-A No. 2009-191239, JP-A No. 2012-158622, JP-A No. 2008-63408, and WO2006/22431 disclose techniques of dispersing nanometer-size inorganic nanoparticles composed of a lamellar clay mineral in a resin composition. However, these techniques respectively have own problems.

The problem of the technique disclosed in JP-A No. 2009-191239 is that in order to increase an ion exchange rate , an organic compound is introduced in ion exchanging in an amount that exceeds ion exchange capacity of a lamellar clay mineral, which results in the presence of a residual organic compound, remaining various metal ions, and the like that are not inserted in an interlamellar space.

Since such an organic compound is kneaded with a resin to produce a resin composition, large amounts of the residual organic compound and various metal ions are present in the resin composition obtained as a final product. Therefore, the resin composition is considered to have a small effect of improving insulation.

In addition, JP-A No. 2009-191239 discloses that an ammonium ion is usually used as the organic compound. In a case in which a large amount of ammonium ions are mixed with an insulating resin composition, the life of the resin composition might be reduced due to moisture absorption by amine. Further, a resin sheet obtained by forming the resin composition into a sheet might become hardened when cured due to catalytic effects of amine.

Meanwhile, the problem of the technique described in JP-A No. 2012-158622 is that a system must be water-based. Due to problems of deactivation of a catalyst in the presence of water, compatibility with a resin, and the like, the technique is considered to be inapplicable for resin composition production.

Further, the problem of the technique described in JP-A No. 2008-63408 is that melt kneading must be conducted at an evaporation temperature of an organic modifier in an interlamellar clay mineral. Therefore, for example, in a case in which a resin sheet is produced thereby, a curing reaction proceeds at the evaporation temperature of the organic modifier, which might cause hardening of a sheet.

Further, the problem of the technique disclosed in WO2006/22431 is that delamination takes place only in a process of compounding, i.e., kneading of a lamellar inorganic compound and a polymer. Therefore, the compounding method and compounding conditions for obtaining sufficient effects are limited.

In addition, JP-A No. 2009-191239, JP-A No. 2012-158622, and JP-A No. 2008-63408 disclose that a lamellar inorganic compound is dispersed in a resin alone. Therefore, they fail to disclose a finding regarding improvement of insulating property by applying a lamellar inorganic compound to an insulating resin composition which is highly filled with an inorganic filler such as alumina that contributes to high thermal conductivity.

As stated above, there is no disclosure of an example which maintains thermal conductivity of an insulating resin composition including a resin and an inorganic filler such as alumina and at the same time improves insulating property of the resin composition. In other words, the development of a thermally-conductive insulating resin composition having high thermal conductance and high insulating reliability has not been achieved so far.

Accordingly, an object of the invention is to provide: a complex of an organic compound and a lamellar inorganic compound in which regular layers of a non-swelling lamellar inorganic compound is expanded via intercalation of the organic compound which improves affinity for a resin; and a method of producing thereof.

Another object of the invention is to provide a delaminated lamellar inorganic compound that has been imparted with a high aspect ratio and a method of producing thereof by a mechanical treatment.

Still another object of the invention is to provide an insulating resin composition, a resin sheet, an insulator, a resin sheet cured product, and a heat dissipating member, which have high insulating voltage resistance.

Solution to Problem

Specific means for achieving the object are described below.

<1> A method of producing a complex of a lamellar inorganic compound and an organic compound, the method comprising:

heat-treating a non-swelling lamellar inorganic compound within a pyrolysis temperature range of the non-swelling lamellar inorganic compound; and
intercalating an organic compound into the non-swelling lamellar inorganic compound in a dispersion liquid in which the heat-treated non-swelling lamellar inorganic compound is dispersed in a medium, thereby inserting the organic compound into an interlamellar space of the non-swelling lamellar inorganic compound,
wherein the non-swelling lamellar inorganic compound comprises unit crystal layers disposed one on another to form a lamellar structure,

the non-swelling lamellar inorganic compound would expand in its c axis direction by from 0.05 Å to 0.20Å when the non-swelling lamellar inorganic compound is heated at a pyrolysis upper limit temperature of the non-swelling lamellar inorganic compound for 1 hour, and

a crystal structure of the unit crystal layers would not change when the non-swelling lamellar inorganic compound is heated at the pyrolysis upper limit temperature for 1 hour.

<2> The method of producing a complex of a lamellar inorganic compound and an organic compound according to <1>, wherein the non-swelling lamellar inorganic compound is mica.

<3> The method of producing a complex of a lamellar inorganic compound and an organic compound according to <1> or <2>, wherein the organic compound is at least one cationic organic compound selected from the group consisting of an amine salt, a phosphonium salt, an imidazolium salt, a pyridinium salt, a sulfonium salt, and an iodonium salt.

<4> The method of producing a complex of a lamellar inorganic compound and an organic compound according to any one of <1> to <3>, wherein a concentration of the organic compound in the dispersion liquid is 0.01 mol/L or more but not more than a solubility of the organic compound, and wherein a content of the non-swelling lamellar inorganic compound in the dispersion liquid is from 0.5% by volume to 50% by volume.

<5> A method of producing a delaminated lamellar inorganic compound, the method comprising:

heat-treating a non-swelling lamellar inorganic compound within a pyrolysis temperature range of the non-swelling lamellar inorganic compound;

intercalating an organic compound into the non-swelling lamellar inorganic compound in a dispersion liquid in which the heat-treated non-swelling lamellar inorganic compound is dispersed in a medium, thereby inserting the organic compound into an interlamellar space of the non-swelling lamellar inorganic compound; and

applying a shear force to the dispersion liquid via a mechanical treatment, thereby delaminating the non-swelling lamellar inorganic compound comprising the intercalation,

wherein the non-swelling lamellar inorganic compound comprises unit crystal layers disposed one on another to form a lamellar structure,

the non-swelling lamellar inorganic compound would expand in its c axis direction by from 0.05Å to 0.20Å when the non-swelling lamellar inorganic compound is heated at a pyrolysis upper limit temperature of the non-swelling lamellar inorganic compound for 1 hour, and

a crystal structure of the unit crystal layers would not change when the non-swelling lamellar inorganic compound is heated at the pyrolysis upper limit temperature for 1 hour.

<6> The method of producing a delaminated lamellar inorganic compound according to <5>, wherein an equilibrium filler density of the dispersion liquid after the application of the shear force to the dispersion liquid is not more than 30% by volume.

<7> The method of producing a delaminated lamellar inorganic compound according to <5> or <6>, wherein an average particle diameter of the delaminated non-swelling lamellar inorganic compound after the application of the shear force to the dispersion liquid is from 50% to 100% of an average particle diameter of the non-swelling lamellar inorganic compound comprising the intercalation before the application of the shear force to the dispersion liquid.

<8> The method of producing a delaminated lamellar inorganic compound according to any one of <5> to <7>, wherein an impingement pressure of the dispersion liquid employed in the mechanical treatment is from 50 MPa to 250 MPa.

<9> A complex of a lamellar inorganic compound and an organic compound,

the complex comprising the organic compound intercalated into a non-swelling lamellar inorganic compound,

wherein the non-swelling lamellar inorganic compound comprises unit crystal layers disposed one on another to form a lamellar structure,

the non-swelling lamellar inorganic compound would expand in its c axis direction by from 0.05Å to 0.20Å when the non-swelling lamellar inorganic compound is heated at a pyrolysis upper limit temperature of the non-swelling lamellar inorganic compound for 1 hour, and

a crystal structure of the unit crystal layers would not change when the non-swelling lamellar inorganic compound is heated at the pyrolysis upper limit temperature for 1 hour.

<10> The complex of a lamellar inorganic compound and an organic compound according to <9>, wherein the organic compound that is intercalated into an interlamellar space of the non-swelling lamellar inorganic compound accounts for from 1% by mass to 40% by mass with respect to 100% by mass of the non-swelling lamellar inorganic compound.

<11> A delaminated lamellar inorganic compound, having an average particle thickness of from 1 nm to 80 nm in its c axis direction.

<12> The delaminated lamellar inorganic compound according to <11>, having an average particle diameter that is from 50% to 100% of an average particle diameter of a non-swelling lamellar inorganic compound comprising intercalation.

<13> An insulating resin composition, comprising a thermosetting resin and an inorganic filler, at least a part of the inorganic filler being the delaminated lamellar inorganic compound according to <11> or <12>.

<14> The insulating resin composition according to <13>, wherein the delaminated lamellar inorganic compound accounts for from 0.5% by volume to 10% by volume of the inorganic filler.

<15> A resin sheet obtained by forming the insulating resin composition according to <13> or <14> into a sheet.

<16> An insulator that is a cured product of the insulating resin composition according to <13> or <14>.

<17> A resin sheet cured product that is a heat-treated product of the resin sheet according to <15>.

<18> A heat dissipating member, comprising: a metal work; and the resin sheet according to <15> or the resin sheet cured product according to <17> disposed on the metal work.

Advantageous Effects of Invention

According to the invention, a complex of a lamellar inorganic compound and an organic compound in which regular lamination in a non-swelling lamellar inorganic compound is expanded via intercalation of the organic compound and which has improved affinity for resin, and a method of producing thereof are provided.

In addition, according to the invention, a delaminated lamellar inorganic compound having a high aspect ratio and a method of producing thereof by a mechanical treatment are provided.

Further, according to the invention, an insulating resin composition, a resin sheet, an insulator, a resin sheet cured product, and a heat dissipating member, which have high insulating voltage resistance, are provided.

BRIEF DESCRIPTION OF DRAWINGS

Fig. 1 illustrates one aspect of the heat dissipating member in the present embodiment.

Fig. 2 is a graph showing XRD measurement results of the sample obtained in Example 1.

Fig. 3 is a photograph showing a state in which a dispersion liquid was left to stand still for 2 weeks, (a) denotes a case in which the dispersion liquid was not subjected to delamination treatment, and (b) denotes a case in which the dispersion liquid was subjected to delamination treatment.

Fig. 4 is a graph showing XRD measurement results of the sample obtained in Comparative Example 2.

Fig. 5 is a graph showing XRD measurement results of the sample obtained in Example 5.

Fig. 6 is a photograph showing an SEM image of a mica powder before delamination treatment.

Fig. 7 is a photograph showing an SEM image of the delaminated compound that was refluxed for 96 hours in Example 5.

Fig. 8 is a graph showing a thickness distribution of the particular complex after delamination treatment obtained in Example 5 and that of a mica powder before delamination treatment obtained in Example 1.

DESCRIPTION OF EMBODIMENTS

Hereinafter, embodiments for carrying out the invention are described in detail. Note that the invention is not limited to the embodiments described below. In the following embodiments, constituent elements (including element steps, etc.) are not essential except a case in which they are particularly specified or considered clearly essential in principle. The same applies to the numerical values and the ranges thereof, which are not intended to limit the invention.

The term "step" used herein refers to not only an independent step but also a step that cannot be clearly distinguished from a different step as long as the object of the step can be achieved.

The numerical range indicated with the word "from ... to ..." includes numerical values described before and after "to" as the minimum and the maximum, respectively.

Regarding the numerical range that is described herein in a step-wise manner, the upper limit or the lower limit of a single numerical range may be replaced by the upper limit or the lower limit of a different numerical range described herein in a step-wise manner. In addition, the upper limit or the lower limit of the numerical range described herein may be replaced by values indicated in the Examples.

Further, in a case in which a plurality of substances corresponding to respective components are present in a composition, the content of each component refers to the total amount of the plurality of substances in the composition, unless otherwise specified.

Furthermore, in a case in which different kinds of particles corresponding to respective components are present in a composition, the particle diameter of each component in the composition refers to a value for a mixture of the different kinds of particles.

The term "resin composition layer" used herein encompasses a configuration of a shape which is formed across the face as well as that on a part thereof when observed in a plan view.

<Complex of Lamellar Inorganic Compound and Organic Compound and Method of Producing thereof>

A method of producing a complex of the lamellar inorganic compound and an organic compound (hereinafter sometimes referred to as a "particular complex") in the present embodiment includes a step of heat-treating a non-swelling lamellar inorganic compound within a pyrolysis temperature range of the non-swelling lamellar inorganic compound and a step of intercalating an organic compound into the non-swelling lamellar inorganic compound in a dispersion liquid in which the heat-treated non-swelling lamellar inorganic compound is dispersed in a medium, thereby inserting the organic compound into an interlamellar space of the non-swelling lamellar inorganic compound. In the method, the non-swelling lamellar inorganic compound includes unit crystal layers disposed one on another to form a lamellar structure, the non-swelling lamellar inorganic compound would expand in its c axis direction by from 0.05Å to 0.20Å when the non-swelling lamellar inorganic compound is heated at a pyrolysis upper limit temperature for 1 hour, and a crystal structure of the unit crystal layers would not change when the non-swelling lamellar inorganic compound is heated at the pyrolysis upper limit temperature for 1 hour.

In addition, as the particular complex in the present embodiment, a compound which includes an organic compound intercalated into a non-swelling lamellar inorganic compound, in which the non-swelling lamellar inorganic compound includes unit crystal layers disposed one on another to form a lamellar structure, the non-swelling lamellar inorganic compound would expand in its c axis direction by from 0.05Å to 0.20Å when the non-swelling lamellar inorganic compound is heated at an upper limit of a pyrolysis temperature thereof for 1 hour, and a crystal structure of the unit crystal layers would not change when the non-swelling lamellar inorganic compound is heated at the pyrolysis upper limit temperature for 1 hour, is used. The particular complex in the present embodiment can be readily obtained by the above-mentioned method of producing thereof.

The present inventors conducted intensive studies to resolve the above-mentioned problems when intercalating an organic compound into a non-swelling lamellar inorganic compound. As a result, the inventors found that an organic compound can be readily intercalated into an interlamellar space of the non-swelling lamellar inorganic compound to form the particular complex by heat-treating a non-swelling lamellar inorganic compound within a pyrolysis temperature range of a non-swelling lamellar inorganic compound to make the non-swelling lamellar inorganic compound expand in its c axis direction. This has led to the completion of the invention.

The particular complex in the present embodiment is useful as a preliminary substance for delamination of a lamellar inorganic compound.

Hereinafter, the particular complex and a method of producing thereof in the present embodiment are specifically explained.

Examples of a non-swelling lamellar inorganic compound used in the present embodiment include mica, kaolinite, and pyrophyllite. Of these, mica that is excellent in insulation is preferable. Examples of non-swelling mica include white mica, black mica, paragonite, margarite, clintonite, anandite, chlorite, phlogopite, lepidolite, muscovite, biotite, taeniolite, and tetrasilicic mica. Note that a non-swelling lamellar inorganic compound used in the present embodiment would expand in its c axis direction by from 0.05Å to 0.20Å when the non-swelling lamellar inorganic compound is heated at an upper limit pyrolysis temperature for 1 hour and a crystal structure of the unit crystal layers would not change when the non-swelling lamellar inorganic compound is heated at the upper limit pyrolysis temperature for 1 hour. In a case in which mica is used as the non-swelling lamellar inorganic compound in the present embodiment, the type of mica is not particularly limited, and it may be a natural product or a product synthesized via hydrothermal synthesis, a melting method, a solid phase method, or the like.

The non-swelling lamellar inorganic compound is a compound in which unit crystal layers stack each other to form a lamellar structure.

A degree of expansion of the non-swelling lamellar inorganic compound in its c axis direction can be measured using an X-ray diffractometer (X-Ray Diffraction, XRD). It is possible to measure a change in distance in the c axis direction by measuring (002) peak shift positions of the non-swelling lamellar inorganic compound before and after heating.

Whether or not there is a change in the crystal structure of the unit crystal layer of the non-swelling lamellar inorganic compound as a result of heating at the upper limit pyrolysis temperature for 1 hour can be checked by determining a change in the crystal structure by measuring the non-swelling lamellar inorganic compound before and after heating by XRD for identification analysis.

The particular complex in the present embodiment can be obtained by intercalating an organic compound into the lamellar inorganic compound. In consideration of affinity for a thermosetting resin in a case in which the delaminated lamellar inorganic compound in the present embodiment described below is used for an insulating resin composition, a substance for intercalation is designated as an organic compound. Type of the organic compound used in the present embodiment is not particularly limited, and it may be at least one cationic organic compound selected from the group consisting of an amine salt, a phosphonium salt, an imidazolium salt, a pyridinium salt, a sulfonium salt, and an iodonium salt.

Examples of the amine salt that can be used in this embodiment include primary to quaternary amine hydrochlorides such as dodecylamine hydrochloride and octadecylamine hydrochloride.

Examples of the phosphonium salt that can be used in this embodiment include a trihexylphosphonium salt.

Examples of the imidazolium salt that can be used in this embodiment include a 1-ethyl-3-methylimidazolium salt.

Examples of the pyridinium salt that can be used in this embodiment include a N-alkylpyridinium salt.

Examples of the sulfonium salt that can be used in this embodiment include a triarylsulfonium salt.

Examples of the iodonium salt that can be used in this embodiment include a N-alkyliodonium salt.

It is possible to extend interlamellar distance and to achieve interlamellar lipophilicity by interlamellarly inserting the organic compound, thereby making a delaminated lamellar inorganic compound prepared from the particular complex to have improved affinity for a rein. This makes it possible to disperse the delaminated lamellar inorganic compound uniformly in the resin.

In the step of the heat-treating a non-swelling lamellar inorganic compound in the method of producing a particular complex in the present embodiment, the non-swelling lamellar inorganic compound is heated within a pyrolysis temperature range of the non-swelling lamellar inorganic compound. When the heating temperature exceeds an upper limit pyrolysis temperature of the non-swelling lamellar inorganic compound, structured water (hydroxyl groups in the crystal structure) of the non-swelling lamellar inorganic compound is eliminated, which tends to cause a change in the crystal structure of the non-swelling lamellar inorganic compound. This is not preferable because it causes the non-swelling lamellar inorganic compound itself to alter. In addition, when the heating temperature is less than the lower limit pyrolysis temperature of the non-swelling lamellar inorganic compound, fluctuation of crystals of the non-swelling lamellar inorganic compound hardly occurs and thus interlamellar distance hardly increases. Thus, intercalation tends not to sufficiently occur. Therefore, the heat treatment temperature of the non-swelling lamellar inorganic compound is determined to fall within a range of pyrolysis temperatures of the non-swelling lamellar inorganic compound. In addition, prior to implementation of the step of heat-treating the non-swelling lamellar inorganic compound, the temperature at which the crystal structure of the non-swelling lamellar inorganic compound changes (i.e., pyrolysis temperature) is examined in advance by thermogravimetric measurement, X-ray diffraction measurement, or the like.

Note that the pyrolysis temperature of the non-swelling lamellar inorganic compound means a temperature range between the upper and lower limits of such temperature.

Details of a method of checking the pyrolysis temperature of the non-swelling lamellar inorganic compound in the present embodiment are as described below. It is possible to measure the pyrolysis temperature by means of thermogravimetric measurement (Thermo Gravimetry, TG) and differential thermal analysis (Differential Thermal Analysis, DTA). It is possible to conveniently measure the pyrolysis temperature based on peak shapes of endothermic and exothermic reactions, peak temperatures of endothermic and exothermic reactions, and the like of DTA obtained when heating the non-swelling lamellar inorganic compound at not less than 500°C. More specifically, a method of observing peak wavelength of structured water, a change in the crystal structure, or the like regarding the heated non-swelling lamellar inorganic compound using an infrared absorption or X-ray diffractometer is preferable.

A reaction in which the organic compound is intercalated into an interlamellar space of the non-swelling lamellar inorganic compound (i.e., a step of interlamellarly inserting the organic compound into the non-swelling lamellar inorganic compound) may be carried out by: making crystals of the non-swelling lamellar inorganic compound unstable via heat treatment to make the non-swelling lamellar inorganic compound to expand in its c axis direction so as to extend an interlamellar space; adding, as a guest compound, an organic compound to a dispersion liquid prepared by dispersing the heat-treated non-swelling lamellar inorganic compound in a medium; and heating and stirring the dispersion liquid. A concentration of the organic compound in the dispersion liquid is preferably a high concentration of not less than 0.01 mol/L in order to increase the number of times of contact between the non-swelling lamellar inorganic compound and the organic compound. However, when the organic compound concentration exceeds a certain level, viscosity of the dispersion liquid might significantly increase. Therefore, it is preferable to adjust the organic compound concentration in the dispersion liquid to a level not more than solubility of the organic compound.

In addition, a content of the non-swelling lamellar inorganic compound in the dispersion liquid is preferably from 0.5% by volume to 50% by volume. When it is not more than 50% by volume, viscosity of the dispersion liquid does not excessively increase, and therefore, reduction of stirring efficiency tends to be suppressed. When it is not less than 0.5% by volume, an amount of the particular complex to be produced tends to be secured at an industrially feasible level.

A medium in which the heat-treated non-swelling lamellar inorganic compound is dispersed is not particularly limited, and it may be a solvent in which the organic compound for intercalation can be dissolved. Specific examples thereof include water and organic solvents such as alcohol. It is also possible to disperse the heat-treated non-swelling lamellar inorganic compound in a medium containing the organic compound for intercalation.

Further, as the temperature upon intercalation reaction increases, the reaction speed increases. Therefore, room temperature (25°C) or higher is preferable.

The organic compound which interlamellarly intercalates into the non-swelling lamellar inorganic compound accounts for preferably from 1% by mass to 40% by mass, more preferably from 1% by mass to 30% by mass, and still more preferably from 1% by mass to 25% by mass with respect to 100% by mass of the non-swelling lamellar inorganic compound. The amount of the intercalated organic compound be from 1% by mass to 40% by mass with respect to 100% by mass of the non-swelling lamellar inorganic compound is preferable in terms of efficient mechanical delamination treatment of the non-swelling lamellar inorganic compound. When the amount of the organic compound for intercalation is not less than 1% by mass, it is possible to extend interlamellar distance in the non-swelling lamellar inorganic compound to fall within a range appropriate for delamination, which causes a tendency to efficiently delaminate the non-swelling lamellar inorganic compound to form it into a nano sheet.

After the step of interlamellarly inserting the organic compound into the non-swelling lamellar inorganic compound, it is possible to redisperse, after eliminating an unreacted organic compound, the intercalated particular complex in a medium.

A method of eliminating an unreacted organic compound is, for example, a washing method including dispersing the intercalated particular complex in water or an organic solvent and collecting it via filtration, filter press, centrifugation or the like. The solvent used for the washing is preferably a solvent for which solubility of the organic compound used for intercalation is high.

Here, an amount of the organic compound used for interlamellar intercalation can be measured by a method such as thermogravimetric measurement (TG) or differential thermal analysis (DTA). It is possible to measure the amount of the organic compound for intercalation into the non-swelling lamellar inorganic compound by measuring a decrease in its mass within a temperature range of from 150°C to 800°C.

<Delaminated Lamellar Inorganic Compound and Method of Producing thereof>

A method of producing a delaminated lamellar inorganic compound (hereinafter sometimes referred to as "delaminated compound") in the present embodiment includes a step of heat-treating a non-swelling lamellar inorganic compound within a pyrolysis temperature range of the non-swelling lamellar inorganic compound, a step of intercalating an organic compound into the non-swelling lamellar inorganic compound in a dispersion liquid in which the heat-treated non-swelling lamellar inorganic compound is dispersed in a medium, thereby inserting the organic compound into an interlamellar space of the non-swelling lamellar inorganic compound, and a step of applying a shear force to the dispersion liquid via a mechanical treatment, thereby delaminating the non-swelling lamellar inorganic compound including the intercalation. In the method, the non-swelling lamellar inorganic compound includes unit crystal layers disposed one on another to form a lamellar structure, the non-swelling lamellar inorganic compound would expand in its c axis direction by from 0.05Å to 0.20Å when the non-swelling lamellar inorganic compound is heated at a pyrolysis upper limit temperature for 1 hour, and a crystal structure of the unit crystal layers would not change when the non-swelling lamellar inorganic compound is heated at the pyrolysis upper limit temperature for 1 hour.

In addition, the delaminated compound in the present embodiment is a delaminated lamellar inorganic compound having an average particle thickness of from 1 nm to 80 nm in its c axis direction. The delaminated compound in the present embodiment can be readily obtained by the above-mentioned method of producing thereof.

The inventors conducted intensive studies in order to solve the problem of reduction in an aspect ratio of a non-swelling lamellar inorganic compound during delamination treatment thereof after intercalating an organic compound thereto. As a result, the inventors found that a delamination product of a non-swelling lamellar inorganic compound having a high aspect ratio can be produced by forming a particular complex in which an organic compound intercalates in a non-swelling lamellar inorganic compound and then mechanically applying high pressure and high shear stress to the particular complex. This has led to the completion of the invention.

A method of applying high shear stress to the particular complex may be a method of applying a shear in a dispersion liquid of the non-swelling lamellar inorganic compound. In consideration of fluid movement, it is preferable to use an apparatus such as a wet jet mill or a high-pressure homogenizer, which can impart a shear flow. In addition, a planetary homogenizer, a high-speed stirrer, a three roll mill, or the like can be employed. In other words, the method may be a method as long as it applies a shear to the particular complex dispersed in a liquid.

Hereinafter, the delaminated compound and the method of producing thereof in the present embodiment are specifically explained.

In the method of producing the delaminated compound in the present embodiment, a step of heat-treating a non-swelling lamellar inorganic compound within a pyrolysis temperature range of a non-swelling lamellar inorganic compound and a step of interlamellarly inserting an organic compound into the non-swelling lamellar inorganic compound are similar to those of the method of producing a particular complex in the present embodiment described above. Therefore, similar materials, treatment conditions, and the like may be applied.

According to the method of producing the delaminated compound in the present embodiment, for example, it is possible to delaminate a lamellar inorganic compound in such a manner that an average particle thickness in the c axis direction thereof falls within a range of from 1nm to 80 nm.

In the step of delaminating the particular complex according to the present embodiment, a concrete method for the delamination is not limited to a wet jet mill or the like. However, there is a tendency that a particular complex is not delaminated but crushed and atomized in a dry crushing method which uses an air flow suction type or collision type jet mill or the like, a ball mill method, or the like, and thus efficient delamination into a nano-sheet form is hardly achieved thereby. Therefore, it is preferable to conduct a step of delamination via a mechanical treatment using an apparatus that can perform shearing at a high-speed in a dispersion liquid such as a wet jet mill or the like. It enables to delaminate the particular complex without crushing it in the longitudinal direction (a axis).

The impingement pressure of the dispersion liquid upon the mechanical treatment in the delamination step is preferably from 50 MPa to 250 MPa, more preferably from 100 MPa to 200 MPa, and still more preferably from 150 MPa to 200 MPa. In addition, the shear speed is preferably from 100 m/s to 400 m/s, more preferably from 180 m/s to 300 m/s, and still more preferably from 200 m/s to 300 m/s. The delaminated compound having a high aspect ratio can be obtained with high productivity by use of this method.

Note that an average particle diameter of the particular complex to be subjected to the delamination step is preferably from 0.01 $\mu$m to 100 $\mu$m before the mechanical treatment. When the average particle diameter of a particular complex before the mechanical treatment is not less than 0.01 $\mu$m, the aspect ratio of the particular complex before the mechanical treatment is not excessively small, and therefore, there is a tendency that delamination via the mechanical treatment in a dispersion liquid easily applies a shear force to facilitate delamination.

Meanwhile, when the average particle diameter of a particular complex before the mechanical treatment is not more than 100 $\mu$m, the particular complex easily disperses in a dispersion liquid before the mechanical treatment and delamination tends to progress by application of a shear force.

Average particle diameters of particles of the particular complex or the like in the present embodiment can be measured using a laser diffraction scattering particle diameter distribution analyzer. An object to be measured is introduced into a dispersion liquid, followed by dispersing using a stirrer or the like. A particle diameter distribution of the object can be measured by measuring a particle diameter distribution of the dispersion liquid. Based on the particle diameter distribution, the average particle diameter can be calculated as a particle diameter corresponding to a cumulative volume of 50% from the small diameter side.

An average particle diameter of the delaminated non-swelling lamellar inorganic compound after application of a shear force to the dispersion liquid (i.e., the delaminated compound) is preferably from 50% to 100%, more preferably from 70% to 100%, and still more preferably from 90% to 100% of an average particle diameter of the non-swelling lamellar inorganic compound that has been subjected to intercalation before applying a shear force to the dispersion liquid (i.e., the particular complex). When the average particle diameter of the delaminated compound is from 50% to 100% of the average particle diameter of the particular complex, it is advantageous in that influence of delamination in the thickness direction (c axis) is greater than influence of destruction in the longitudinal direction (a axis), which means that reduction of the aspect ratio is suppressed.

The average particle thickness of the delaminated compound in its c axis direction in the present embodiment can be measured by the following method using a scanning electron microscope (Scanning Electron Microscope, SEM). First, the delaminated compound and a dispersion medium are mixed to prepare a dispersion slurry. The prepared dispersion slurry is poured into a mold to obtain a disk-shaped delaminated compound compact. As delaminated particles have a large aspect ratio, they are layered in the thickness direction in such a disk-shaped delaminated compound compact. The thickness of the delaminated compound can be measured by observing the disk-shaped delaminated compound compact by SEM from the lateral direction. It is possible to make a thickness distribution chart by measuring the thickness of the delaminated compound at randomly selected 200 or more sites and conducting image analysis. A cumulative 50% thickness ($T_{50}$) in the thickness distribution is designated as the average particle thickness in the c axis direction.

A thickness of the delaminated compound after the mechanical treatment can be specified by measurement of an equilibrium filler density, in addition to the thickness distribution obtained using a scanning electron microscope (SEM). Here, the term "equilibrium filler density" refers to bulk density of the delaminated compound in a dispersion liquid thereof when equilibrium is achieved in the dispersion liquid. It is an index which is expressed in terms of a volume percentage and which indicates a degree of presence of the delaminated compound in a certain volume of the dispersion liquid after dispersing and stabilization. Since the particular complex that is a laminated body is subjected to delamination, a sediment thickness of the delaminated compound in a dispersion liquid increases as a result of the delamination, and thus the density of the delaminated compound therein decreases. In other words, the mechanical treatment causes increase ina number of particles and decrease in the equilibrium filler density even when the filling volume (volume content) is maintained. It is preferable for the equilibrium filler density to decrease. In the present embodiment, it is preferably not more than 30% by volume. When the equilibrium filler density is not more than 30% by volume, it can be said that delamination proceeded efficiently.

The equilibrium filler density can be measured by the following method.

A certain amount of the delaminated compound slurry after the mechanical treatment is added to a test tube and left to stand still at room temperature (25°C) for 2 weeks. Calculation of the equilibrium filler density according to Equation (3) is enabled by measuring the sediment height of the delaminated compound that has been left to stand still for 2 weeks.

$$\text{Equilibrium filling density (\%)} = \text{Delaminated compound concentration in slurry} /$$

$$\{(\text{Delaminated compound sediment height}) / \text{Slurry height})\} \qquad (3)$$

The delaminated compound obtained via the delamination step may be obtained as a powdered sample after drying or it may be obtained as a slurry sample in liquid. Alternatively, it may be stored together with an organic dispersing agent, a thickening agent, and the like in liquid. It is possible to use such delaminated compound in various forms in accordance with the intended use.

The use of a particular complex in the present embodiment enables to provide the delaminated compound without a decrease in its length in the longitudinal direction (a axis). Therefore, the aspect ratio of the delaminated compound increases. As a result of compounding of a thermoplastic resin or a thermosetting resin and the delaminated compound having a high aspect ratio, it becomes possible to effectively exhibit properties of the non-swelling lamellar inorganic compound such as insulating, voltage resistance, and heat resistance properties. Further, the delamination enables to suppress an amount of the delaminated compound used as an inorganic filler filled into a resin. Therefore, it is possible to resolve various problems caused by an increase in the degree of filling with an inorganic filler, for example, deterioration of formability due to a decrease in fluidity, increases in material cost and manufacturing cost due to the use of a large amount of an inorganic filler, an increase in the weight of materials and members, and deterioration of insulating characteristics due to defects. This achieves an improvement in voltage resistance. Further, the delaminated compound of the present embodiment can be provided in the form of either a slurry or a powder. Therefore, the delaminated compound can be readily incorporated into a manufacturing process of various nanocomposites.

For example, compounding thereof with a thermosetting resin such as an epoxy resin will lead to a development of an insulating resin material which is excellent in insulating property, voltage resistance, heat resistance and the like.

<Insulating Resin Composition>

An insulating resin composition in the present embodiment contains a thermosetting resin and an inorganic filler, and at least a part of the inorganic filler is the delaminated compound of the present embodiment.

A conventionally used insulating resin composition containing round-shaped alumina or the like, which has small anisotropy and which contributes to the improvement in thermal conductivity, has a short dielectric breakdown path, which causes reduction in the dielectric breakdown voltage of a resin composition. Therefore, it has been required to add a scale-shaped filler in some cases.

However, when a scale-shaped filler having a size in a micron order or greater is added to an insulating resin composition, a longitudinal direction of the scale-shaped filler tends to be oriented in a direction perpendicular to a thickness direction of the resin composition, which tends to result in a decrease in thermal conductivity in the thickness direction of the resin composition. It is considered that use of nanometer-size inorganic nanoparticles is more effective for preventing the phenomenon.

Nano-size lamellar inorganic compounds such as BN and mica can be considered as the inorganic nanoparticles. However, BN has few surface functional groups, which means poor affinity with a resin, and accordingly, it tends to cause void formation inside of a resin composition, which may reduce insulation property.

The inventors conducted intensive studies in order to solve the above problem. As a result, the inventors found that it becomes possible to produce an insulating resin composition having high insulating reliability by using a lamellar inorganic compound which has been delaminated to have a specific thickness (the delaminated compound of the present embodiment). Thereby, it became possible to realize a resin composition, a resin sheet, an insulator, and a resin sheet cured product, which have high insulating voltage resistance, and heat dissipating members using the same by more versatile and convenient steps than before.

The delaminated compound of the present embodiment is excellent not only in an electric insulating property but also in properties such as heat resistance and chemical resistance properties, and it is further excellent in cost performance.

Hereinafter, components and the like of an insulating resin composition of the present embodiment are explained.

(Thermosetting Resin)

An insulating resin composition in the present embodiment contains at least one thermosetting resin. Examples of the thermosetting resin include an epoxy resin, an oxazine resin, a bismaleimide resin, a phenol resin, an unsaturated polyester resin, and a silicone resin. In view of electric insulation, an epoxy resin is preferable.

The epoxy resin used in the present embodiment is not particularly limited. Examples thereof include a bisphenol F type epoxy resin, a bisphenol S type epoxy resin, a phenol novolac type epoxy resin, a cresol novolac type epoxy resin, a naphthalene type epoxy resin, and a cyclic aliphatic epoxy resin. Of these, in view of achievement of high thermal conductivity, it is preferable to use an epoxy resin having an intramolecular mesogen skeleton, that is a structure of self-ordering groups, such as biphenyl groups. Such epoxy resin having an intramolecular mesogen skeleton is disclosed in, for example, JP-A No. 2005-206814. Example of the above-mentioned epoxy resin include 1-{(3-methyl-4-oxiranylmethoxy)phenyl}-4-(4-oxiranylmethoxyphenyl)-1-cyclohexene, 1-{(2-methyl-4-oxiranylmethoxy)phenyl}-4-(4-oxiranylmethoxyphenyl)-1-cyclohexene and 1-{(3-ethyl-4-oxiranylmethoxy)phenyl}-4-(4-oxiranylmethoxyphenyl)-1-cyclohexene.

A content of a thermosetting resin in an insulating resin composition in the present embodiment is not particularly limited. For example, it can be from 1% by mass to 50% by mass and it is preferably from 1% by mass to 10% by mass with respect to of a solid content of the insulating resin composition. When the content of the thermosetting resin falls within such range, adhesiveness and thermal conductivity can be further improved. Note that the solid content of the insulating resin composition means residues left after eliminating a volatile component from the insulating resin composition.

(Inorganic Filler)

The insulating resin composition in the present embodiment contains an inorganic filler. In the present embodiment, at least a part of the inorganic filler is composed of the delaminated compound in the present embodiment.

It is preferable to set a percentage of the delaminated compound contained in the inorganic filler to from 0.5% by volume to 10% by volume. In a case in which the content of the delaminated compound is not less than 0.5% by volume, insulating property of the insulating resin composition tends to be improved. Meanwhile, in a case in which the content of the delaminated compound is not more than 10% by volume, thermal conductivity of the insulating resin composition tends be improved.

In a case in which an inorganic filler other than the delaminated compound is used in the present embodiment, such an inorganic filler other than the delaminated compound is not particularly limited, and compounds conventionally known in the art can be used. Examples thereof include aluminum oxide (alumina), magnesium oxide, aluminum nitride, boron nitride, silicone nitride, silicone dioxide, aluminum hydroxide, and barium sulfate.

In a case in which an inorganic filler other than a delaminated compound is used in the present embodiment, the inorganic filler other than the delaminated compound may be used singly, or in combination/mixture of two or more kinds thereof. Alternatively, it is also possible to use inorganic fillers having different particle diameters in combination. An embodiment in which a combination of inorganic fillers having different particle diameters is used is preferable because it is considered that an inorganic filler having a small particle diameter enters gaps in an inorganic filler having a large particle diameter, which facilitates to increase filling of the inorganic filler, thereby achieves high thermal conductivity with good efficiency.

In view of thermal conductance, an average particle diameter (D50) of the inorganic filler is preferably from 0.1 $\mu$m to 100 $\mu$m, and more preferably from 0.1 $\mu$m to 70 $\mu$m.

A method of measuring the average particle diameter of the inorganic filler in the present embodiment is the same as in the case of that for particles of the particular complex or the like.

In one embodiment of the present embodiment, it is preferable to use alumina as an inorganic filler and more preferable to use any combination of inorganic fillers of alumina having different particle diameters.

A content of all inorganic fillers in an insulating resin composition in the present embodiment is not particularly limited. It is particularly preferably from 30% by volume to 95% by volume with respect to a total volume of a solid content of the insulating resin composition. In view of improvement of thermal conductivity, it is more preferably from 45% by volume to 90% by volume. In view of further improvement of thermal conductivity, it is still more preferably from 70% by volume to 90% by volume. When the total content of inorganic filler is not less than 30% by volume of a total volume of a solid content of the insulating resin composition, thermal conductivity of the insulating resin composition tends to further increase. In addition, when the total content of inorganic filler is not more than 95% by volume with respect to a total volume of a solid content of the insulating resin composition, formability of the insulating resin composition tends to further improve.

Note that the total volume of a solid content of the insulating resin composition means the total volume of nonvolatile components among components that configure the insulating resin composition.

(Curing Agent)

It is preferable that the insulating resin composition contains at least one curing agent. The curing agent is not particularly limited and it may be appropriately selected depending on a type of a thermosetting resins. In particular, in a case in which a thermosetting resin is an epoxy resin, it is possible to appropriately select a curing agent from curing agents generally used for epoxy resins and use it. Specific examples thereof include: an amine-based curing agent such as dicyandiamide or an aromatic diamine; and a phenol-based curing agent such as a phenol novolac resin, a cresol novolac resin, and a catechol resorcinol novolac resin. Of these, in view of improvement of thermal conductivity, the curing agent is preferably a phenol-based curing agent, and more preferably a phenol-based curing agent containing a structure unit derived from a bifunctional phenollic compound such as catechol, resorcinol, or p-hydroquinone.

In a case in which the insulating resin composition contains the curing agent, a content of the curing agent in the insulating resin composition is not particularly limited. For example, the content of the curing agent for 1 equivalent of an epoxy resin can be from 0.1 equivalents to 2.0 equivalents. In view of improvement of flexibility, it is preferably from 0.5 equivalents to 1.5 equivalents. In view of high thermal conductivity, it is more preferably from 0.8 equivalents to 1.1 equivalents.

When the content of the curing agent falls within the above range, there is a tendency that thermal conductivity can be further improved.

(Curing Catalyst)

It is preferable that the insulating resin composition contains at least one curing catalyst. The curing catalyst is not particularly limited, and it may be appropriately selected from conventionally used curing catalysts depending on a type of thermosetting resin and used. In a case in which the thermosetting resin is an epoxy resin, specific examples of the curing catalyst include triphenylphosphine, 2-ethyl-4-methylimidazole, a boron trifluoride amine complex, and 1-benzyl-2-methylimidazole. Of these, it is preferable to use triphenylphosphine in view of achievement of high thermal conductivity.

In a case in which the insulating resin composition contains the curing catalyst, a content of the curing catalyst in the insulating resin composition is not particularly limited. In the insulating resin, the content of the curing catalyst composition with respect to, for example, an epoxy resin, can be preferably from 0.1% by mass to 2.0% by mass, and more preferably from 0.5% by mass to 1.5% by mass.

When the content of the curing catalyst falls within the above range, there is a tendency that thermal conductivity can be further improved.

(Coupling Agent)

It is preferable that the insulating resin composition contains at least one coupling agent. The coupling agent may be contained for the purpose of, for example, surface treatment of an inorganic filler.

The coupling agent is not particularly limited, and it may be appropriately selected from conventionally used coupling agents. Specific examples thereof include methyltrimethoxysilane (manufactured by Shin-Etsu Chemical Co., Ltd., available under the product name "KBM-13"), 3-mercaptopropyltrimethoxysilane (manufactured by Shin-Etsu Chemical Co., Ltd., available under the product name "KBM-803"), 3-triethoxysilyl-N-(1,3-dimethyl-butylidene)propylamine (manufactured by Shin-Etsu Chemical Co., Ltd., available under the product name "KBE-9103"), N-phenyl-3-aminopropyltrimethoxysilane (manufactured by Shin-Etsu Chemical Co., Ltd., available under the product name "KBM-573"), 3-aminopropyltrimethoxysilane (manufactured by Shin-Etsu Chemical Co., Ltd., available under the product name "KBM-903"), and 3-glycidyloxypropyltrimethoxysilane (manufactured by Shin-Etsu Chemical Co., Ltd., available under the product name "KBM-403"). Of these, in view of achievement of high thermal conductivity, N-phenyl-3-aminopropyltrimethoxysilane is preferable.

In a case in which the insulating resin composition contains a coupling agent, the content of the coupling agent in the insulating resin composition is not particularly limited. The content of the coupling agent in the insulating resin composition can be set to, for example, from 0.05% by mass to 1.0% and it is preferably from 0.1% by mass to 0.5% by mass by mass with respect to that of the inorganic filler.

When the content of the coupling agent falls within the above-mentioned range, there is a tendency that thermal conductivity can be further improved.

(Solvents)

The insulating resin composition may contain at least one solvent. The solvent is not particularly limited as long as it does not inhibit a curing reaction of the resin composition. It may be appropriately selected from conventionally used organic solvents and used. Specific examples thereof include: a ketone solvent such as methylethylketone and cyclohexanone; and an alcohol solvent such as cyclohexanol.

In a case in which the insulating resin composition contains the solvent, a content of the solvent in the insulating resin composition is not particularly limited, and it can be appropriately selected depending on the coating suitability of the resin composition, etc.

(Additive)

The insulating resin composition may further contain additives other than the curing catalyst and the solvent as described above, if necessary. Examples of such other additive include elastomers that can improve a delamination property and dispersibility of the delaminated compound. Other examples include various additives generally used for resin compositions, such as antioxidants, anti-aging agents, stabilizers, flame retardants, and thickening agents. In a case in which the insulating resin composition further contains additives, the contents of these additives are not particularly limited as long as the effects of the invention is not impaired.

<Resin sheet>

A resin sheet in the present embodiment is obtained by forming the insulating resin composition in the present embodiment into a sheet shape.

The resin sheet in the present embodiment is not particularly limited as long as it is obtained by forming the insulating resin composition in the present embodiment into a sheet shape. It is preferable for the resin sheet in the present embodiment to be a so-called B stage sheet, that is further heat-treated to be in a semi-cured state (B stage state).

The term "B stage" used herein is specified based on the definition of JIS K6900:1994.

The resin sheet can be produced in the following manner, for example. A resin composition layer can be obtained by applying, on a mold-releasing film such as a PET (polyethyleneterephthalate) film, an insulating resin composition which is in a form of varnish and to which a solvent such as methylethylketone or cyclohexanone has been added if necessary, and then drying the coating if necessary.

The application can be conducted by a conventionally known method. Specific examples of a method of the application include a comma coating method, a die coating method, a lip coating method, and a gravure coating method. As a method of application for forming the resin composition layer having a certain thickness, a comma coating method in which a subject to be coated is made to pass through a gap, a die coating method in which resin varnish is applied from a nozzle while controlling its flow rate, or the like can be applied. For example, in a case in which a thickness of a resin composition layer before drying is from 50 $\mu$m to 500 $\mu$m, it is preferable to employ a comma coating method.

Thickness of the resin sheet can be appropriately selected depending on a purpose. For example, it can be from 50 $\mu$m to 300 $\mu$m. In view of thermal conductivity and sheet flexibility, it is preferably from 60 $\mu$m to 250 $\mu$m. In addition, the resin sheet can be prepared by heat pressing two or more resin composition layers being stacked.

<Insulator>

The insulator in the present embodiment is a cured product of the insulating resin composition in the present embodiment. The insulator in the present embodiment can be produced by a production method in a similar manner to a case in which a usual casting insulator resin is used, for example, by injecting the insulating resin composition in the present embodiment into a metal mold. By using the insulating resin composition in the present embodiment, it is possible to obtain an insulator having high insulating voltage resistance, compared with the use of epoxy resins used as conventional casting resins. Examples of such insulator include an insulating spacer, an insulating rod, and a molded insulating part.

<Resin Sheet Cured Product>

The resin sheet cured product in the present embodiment is a heat-treated product of the resin sheet in the present embodiment.

The resin sheet cured product in the present embodiment may be obtained by curing the insulating resin composition in the present embodiment via heat treatment. A method of curing the insulating resin composition can be appropriately selected depending on a configuration of the insulating resin composition, a purpose of the resin sheet cured product, or the like. A method of curing the insulating resin composition is preferably heating pressurization treatment. Conditions of heating and pressurization treatment are preferably, for example, a heating temperature of from 80°C to 250°C and a pressure of from 0.5 MPa to 8.0 MPa, and more preferably a heating temperature of from 130°C to 230°C and a pressure of from 1.5 MPa to 5.0 MPa.

A treatment time for the heating and pressurization treatment can be appropriately selected depending on the heating temperature and the like. For example, it can be from 2 hours to 8 hours, and more preferably from 4 hours to 6 hours.

The heating and pressurization treatment may be conducted once or it may be conducted twice or more while changing the heating temperature or the like.

<Heat Dissipating Member>

A heat dissipating member in the present embodiment has a metal work and the resin sheet in the present embodiment or the resin sheet cured product in the present embodiment which is disposed on the metal work.

The term "metal work" used herein refers to a molded article containing a metal material which can function as a heat dissipating member such as a substrate or a fin. In one aspect of the present embodiment, the metal work is preferably a substrate formed of a metal selected from various metals such as Al (aluminum) and Cu (copper).

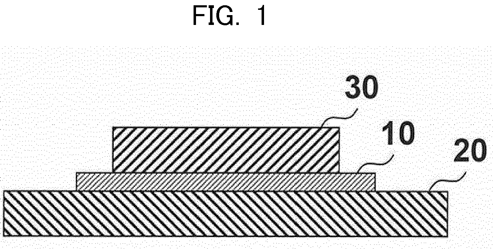

As one aspect of the heat dissipating member of the present embodiment, Fig. 1 exemplarily illustrates a heat dissipating member using a resin sheet obtained by forming the insulating resin composition into a sheet shape.

In Fig. 1, a resin sheet 10 is positioned between a first metal work 20 composed of, for example, Al (aluminum), and a second metal work 30 composed of, for example, Cu (copper), and one side thereof is in contact with a surface of the metal work 20 and the other side there of is in contact with a surface of the metal work 30 surface.

As the resin sheet 10 has high insulating voltage resistance, insulation between the first metal work 20 and the second metal work 30 can be secured even in a case in which, for example, there is a significant potential difference generated between the first metal work 20 and the second metal work 30.

EXAMPLES

Hereinafter, the invention is explained in more detail based on the Examples below. However, the invention is not limited to the Examples.

[Example 1]

Muscovite originating from India (SJ-005 manufactured by Yamaguchi Mica Co., Ltd.; pyrolysis temperature: from 600°C to 800°C) was used as a non-swelling lamellar inorganic compound. SJ-005 expands by 0.09Å in its c axis direction when the non-swelling lamellar inorganic compound is heated at 800°C for 1 hour. As a result of powder X-ray diffraction measurement (RINT-2550 manufactured by Rigaku Corporation), the basal spacing ($d_{002}$) was 9.98Å. In addition, as a result of measurement of particle diameter distribution using a laser diffraction particle diameter distribution analyzer (LA-920 manufactured by HORIBA, Ltd.), an average particle diameter was 5.38 $\mu$m. Muscovite (0.2 g) and sodium carbonate (2 g) were melted at 950°C for 30 minutes, followed by hydrogen fluoride (HF) treatment for removal of Si. Then, 5 mL of 18% by mass hydrochloric acid and 15 mL of water were added to the residue. The mixture was heated on a hot plate (125°C) to be dissolved, and then, water was added thereto to result in an amount of approximately 100 g. The obtained mixture was diluted 10-fold, followed by quantitative analysis by ICP optical emission spectrometry (ICP-OES). As a result, the chemical composition of this sample was found to be $(K_{0.97}Ca_{0.01})(Al_{1.75}Mg_{0.11}Fe^{3+}_{0.11})(Si_{3.21}Al_{0.79})O_{10}(OH)_2$.

The muscovite powder was placed in a crucible and heat-treated in an electric furnace (SB2025D manufactured by MOTOYAMA) at 800°C for 1 hour. A 0.5M aqueous solution in which dodecylamine hydrochloride (DDA-HCl manufactured by Tokyo Chemical Industry Co., Ltd.) is dissolved as an organic compound in 200 mL of distilled water is mixed with 11.2 g of the heat-treated muscovite powder. This liquid mixture (dispersion liquid) was stirred with reflux at 120°C for 24 hours and then washed with water and ethanol (manufactured by Wako Pure Chemical Industries, Ltd.). Thus, a particular complex was prepared.

A content of the muscovite powder in the liquid mixture (dispersion liquid) was 2% by volume.

An average particle diameter of the particular complex was 4.50 $\mu$m.

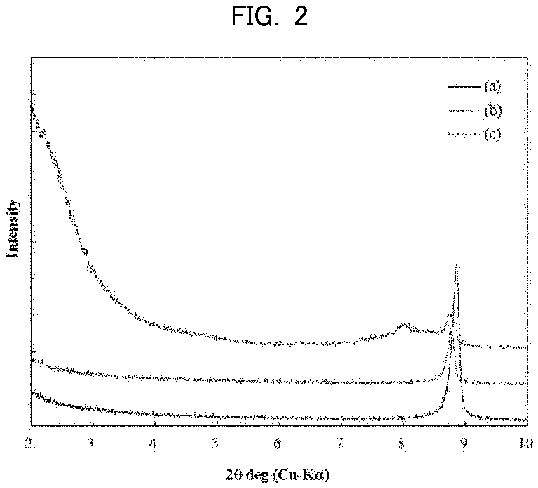

Fig. 2 shows XRD results of the obtained sample. Muscovite was observed to have a sharp 002 reflection (9.98Å) at $2\theta = 8.86°$ (curve (a)). The heat-treated muscovite showed a slight shift to the low angle side (10.07Å) (curve (b)). The sample that had been stirred and refluxed for 24 hours was observed to have a 002 reflection with a decreased peak strength and a peak increase at $2\theta =$ from 3° to 2°. It was revealed that the sample was obtained as a mixture of an intercalated layer and a partial, non-swelled layer (curve (c)).

Note that in Fig. 2, the lowest spectrum corresponds to curve (a), the second lowest spectrum corresponds to curve (b), and the highest spectrum corresponds to curve (c).

In order to calculate a content of dodecylamine hydrochloride in the particular complex subjected to intercalation, a mass decrease was measured at from 150°C to 800°C with a temperature increase speed of 10°C /minute using a thermogravimetric measurement differential thermal analyzer (TG-8120 manufactured by Rigaku Corporation). As a result, the content of dodecylamine hydrochloride was 2.97% by mass.

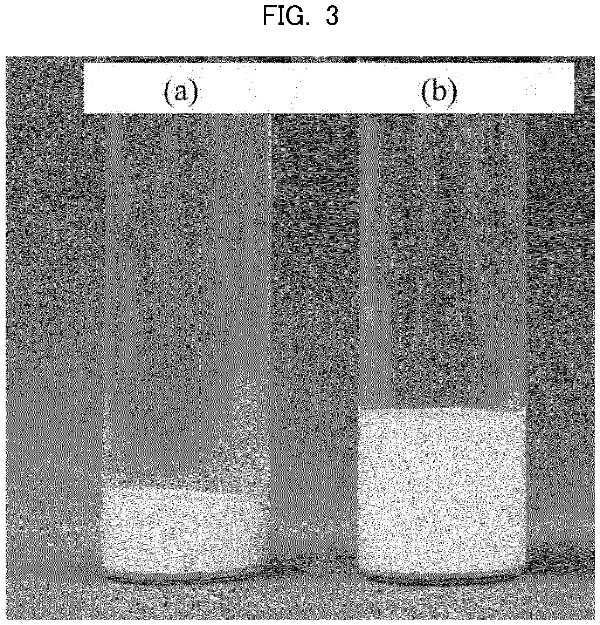

Next, in order to mechanically delaminate the particular complex subjected to intercalation, 11.2 g of the particular complex was dispersed in 200 mL of methylethylketone (MEK), thereby preparing a dispersion liquid. The dispersion liquid was treated by applying a high-speed shear at a shear speed of 280 m/s under a high-pressure of 180 MPa using a wet jet mill, thereby delaminating the particular complex in the dispersion liquid. Thus, a nano-mica sheet dispersion liquid having a high aspect ratio was obtained. In Fig. 3, (a) indicates a state of the dispersion liquid which was left to stand still for 2 weeks without implementation of delamination treatment, and (b) indicates a state of the dispersion liquid which was left to stand still for 2 weeks after implementation of delamination treatment. As a result of calculation of equilibrium filler density based on (a) and (b) in Fig. 3, the equilibrium filler density was 4.25% by volume after delamination treatment, while it was 14.7% by volume before delamination treatment. It was revealed that the particular complex was delaminated in the liquid after delamination treatment.

[Comparative Example 1]

A particular complex was prepared in the same manner as Example 1 except that heat treatment of muscovite was not conducted. As a result of XRD measurement, a very strong basal reflection was observed at 9.98Å. This revealed that dodecylamine hydrochloride was not interlamellarly intercalated into muscovite. In addition, the content of dodecylamine hydrochloride was 0.95% by mass, which is considered to be an amount of organic material adsorbed to a surface of mica. Further, the equilibrium filler density after delamination treatment was 5.90% by volume.

[Comparative Example 2]

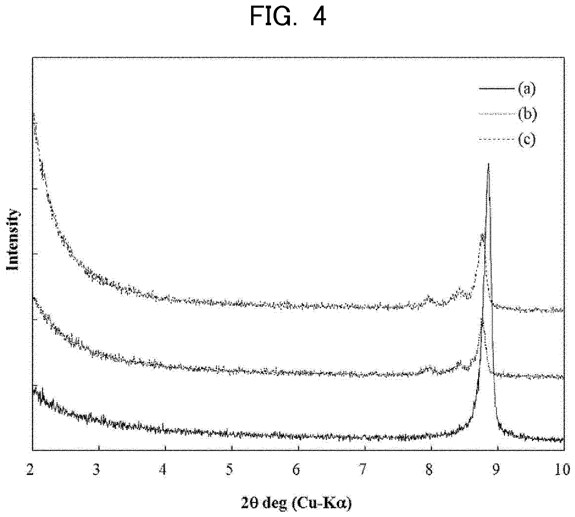

A particular complex was prepared in the same manner as Example 1 except that the temperature of heat treatment of muscovite was set to 1000°C. As a result of XRD measurement (Fig. 4), compared with the muscovite powder (curve (a)), a decrease in peak strength of a 002 reflection was observed even in a case in which only heat treatment was performed (curve (b)). However, before and after intercalation, little peak strength change was observed (curve (c)). In addition, the content of dodecylamine hydrochloride was 0.68% by mass. The equilibrium filler density after delamination treatment was 8.79% by volume.

Note that the lowest spectrum corresponds to curve (a), the second lowest spectrum corresponds to curve (b), and the highest spectrum corresponds to curve (c) in Fig. 4.

[Example 2]

A particular complex was prepared in the same manner as Example 1 except that the concentration of dodecylamine hydrochloride was set to 1.0 M or 2.0 M. As a result of XRD measurement, a 002 reflection with a decrease in peak strength and a peak increase at $2\theta$ = from 3° to 2° were observed at each concentration, revealing that intercalation proceeded. The content of dodecylamine hydrochloride was 2.59% by mass at 1.0 M and 1.02% by mass at 2.0 M. In addition, the equilibrium filler density after delamination treatment was 3.44% by volume at 1.0 M and 4.43% by volume at 2.0 M.

[Example 3]

600 mL of ethanol was stirred at 30°C, during which 200 g of octadecylamine (manufactured by Tokyo Chemical Industry Co., Ltd.) was heat-dissolved therein, and 125 mL of concentrated hydrochloric acid (manufactured by Wako Pure Chemical Industries, Ltd.) was added thereto to cause a reaction to proceed for 3 hours. The solvent was distilled away using an evaporator, followed by recrystallization with ethanol. The resulting crystals were collected and dried under reduced pressure, thereby obtaining octadecylamine hydrochloride (ODA-HCl).

A particular complex was prepared in the same manner as Example 1 using, as an organic compound, the above octadecylamine hydrochloride, distilled water, and muscovite powder. As a result of XRD measurement, a decrease in peak strength of a 002 reflection, a moderate broad peak at $2\theta$ = from 3.5° to 6.5°, and a very large peak increase at $2\theta$ = from 3° to 2° were observed, thereby progress in intercalation being recognized. In addition, the content of hydrochloride was 13.80% by mass. The equilibrium filler density after delamination treatment was 2.53% by volume.

[Example 4]

A particular complex was prepared in the same manner as Example 1 except that reflux time was set to 96 hours. As a result of XRD measurement, a decrease in peak strength of a 002 reflection and a peak increase at $2\theta$ = from 3° to 2° were observed. In addition, the content of dodecylamine hydrochloride was 3.18% by mass. The equilibrium filler density after delamination treatment was 3.81% by volume.

[Example 5]

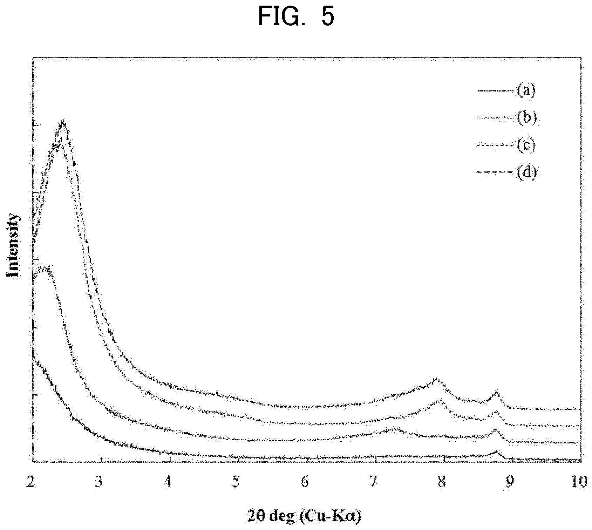

Reflux was carried out for 24 hours in the same manner as in Example 1. Then, muscovite that precipitated by centrifugation was collected, and mixed again with an equivalent amount of a dodecylamine hydrochloride aqueous solution. Mixing, 24-hour reflux, and centrifugation were repeated so that reflux time was adjusted to 48 hours, 72 hours, or 96 hours in total to prepare particular complexes were prepared respectively. Fig. 5 shows XRD results for the obtained sample. Unlike the case of 24 hours (curve (a)) in Example 1, as the reaction time increased to 48 hours (curve (b)), 72 hours (curve (c)), and 96 hours (curve (d)), the peak of the intercalated layer shifted to the higher angle side, and the peak was more sharpened. This suggests that a non-swelled layer shifted to an intercalated layer. In addition, the content of dodecylamine hydrochloride was 4.46% by mass (48 hours), 5.31% by mass (72 hours), and 5.67% by mass (96 hours). There was an increase in intercalation quantity with an increase in the reaction time with solution replacement. The equilibrium filler density after delamination treatment was 2.71% by volume (48 hours), 2.53% by volume (72 hours), and 2.19% by volume (96 hours).
Note that the lowest spectrum corresponds to curve (a), the second lowest spectrum corresponds to curve (b), the second highest spectrum corresponds to curve (c), and the highest spectrum corresponds to curve (d) in Fig 5.
Table 1 lists the composition, organic compound content, and equilibrium filler density for the particular complexes prepared in Examples 1 to 5 and Comparative Examples 1 and 2.

[Table 1]

| | | Heating temperature (°C) | Intercalation | | | | Organic compound content (% by mass) | Equilibrium filler density (% by volume) |
|---|---|---|---|---|---|---|---|---|
| | | | Organic compound | | Time (h) | Solution replacement | | |
| | | | Type | Concentration (M) | | | | |
| Example | 1 | 800 | DDA-HCl | 0.5 | 24 | - | 2.97 | 4.25 |
| | 2 | 800 | DDA-HCl | 1.0 | 24 | - | 2.59 | 3.44 |
| | | 800 | DDA-HCl | 2.0 | 24 | - | 1.02 | 4.43 |
| | 3 | 800 | ODA-HCl | 0.5 | 24 | - | 13.80 | 2.53 |
| | 4 | 800 | DDA-HCl | 0.5 | 96 | None | 3.18 | 3.81 |
| | 5 | 800 | DDA-HCl | 0.5 | 48 | Done | 4.46 | 2.71 |
| | | 800 | DDA-HCl | 0.5 | 72 | Done | 5.31 | 2.53 |
| | | 800 | DDA-HCl | 0.5 | 96 | Done | 5.67 | 2.19 |
| Comparative Example | 1 | None | DDA-HCl | 0.5 | 24 | - | 0.95 | 5.90 |
| | 2 | 1000 | DDA-HCl | 0.5 | 24 | - | 0.68 | 8.79 |

As a result of comparison of Example 1, Comparative Example 1, and Comparative Example 2 in terms of the content of dodecylamine hydrochloride and the equilibrium filler density (Table 1), it was found that when the heating temperature was 800°C, the content of dodecylamine hydrochloride increased while the equilibrium filler density decreased. This indicates that an increase in intercalation quantity caused expansion of interlamellar space, resulting in efficient progress in delamination. Meanwhile, in a case in which heating was not conducted or the heating temperature was 1000°C, progress in intercalation was not observed. This was because as stated above, in a case in which heating was not conducted, there was no fluctuation in the crystal structure of mica, resulting in no expansion of interlamellar space. It was also because in a case in which the heating temperature was 1000°C, elimination of structured water caused pyrolysis, which largely damaged the crystal structure of mica and caused mica itself to alter, resulting in no progress in intercalation.

As a result of comparison of Example 1 and Example 2 in terms of the content of dodecylamine hydrochloride and the equilibrium filler density (Table 1), it was found that the intercalation quantity decreased at a concentration of 2.0 M. This was because the solution viscosity (viscosity of 1.7 mPa·s at 0.5 M, 12 mPa·s at 1.0 M, and 758 mPa·s at 2.0 M at 60°C) was high, resulting in reduction of stirring (contact) efficiency.

Further, as a result of comparison of Example 1, Example 4, and Example 5 in terms of the content of dodecylamine hydrochloride and the equilibrium filler density (Table 1), it was recognized that as intercalation time was prolonged, the content of dodecylamine hydrochloride increased while the equilibrium filler density decreased. In addition, it was shown that intercalation efficiency can be improved by replacing a reaction solution every 24 hours.

[Example 6]

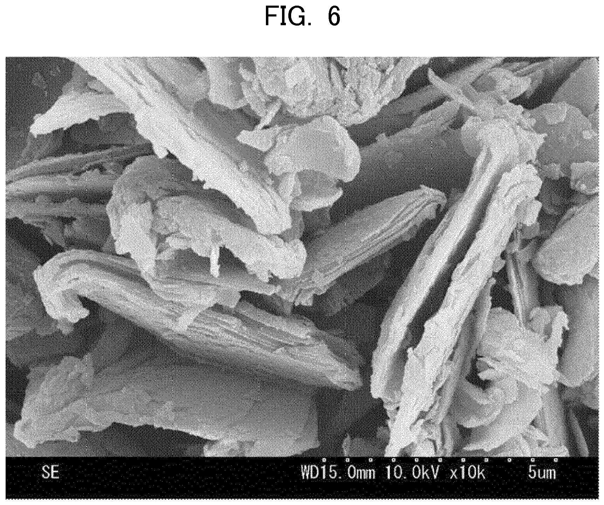

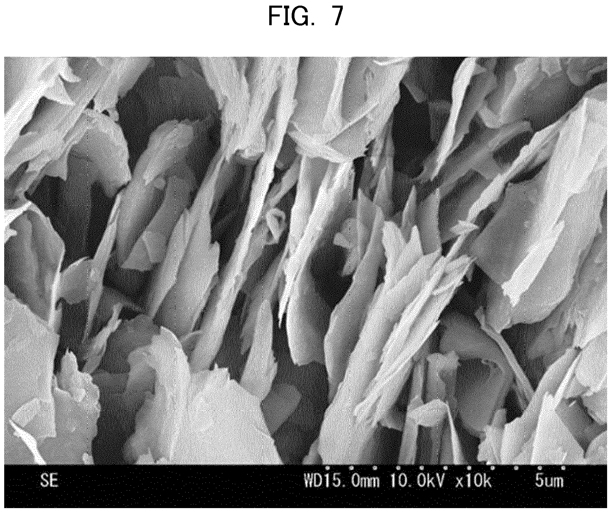

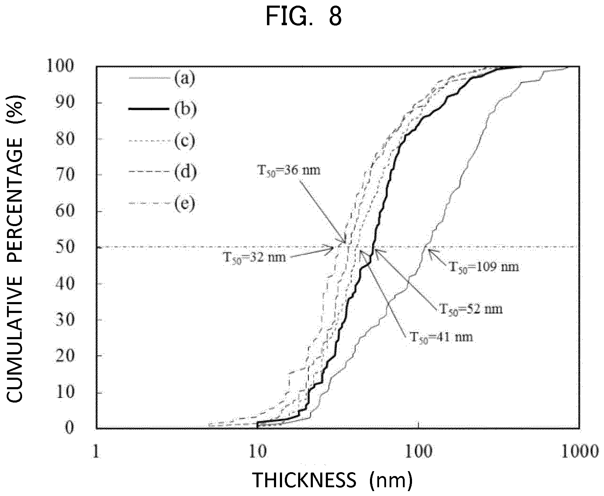

Thickness in the c axis direction was determined by the following method using the particular complex (delaminated compound) after delamination treatment and a mica powder before delamination treatment of Examples 1 and 5. First, the delaminated compound was powderized via lyophilization. Next, the delaminated compound and the mica powder before delamination treatment were each mixed with water, and a dispersing agent was added thereto, thereby preparing dispersion slurries. A silicon mold was placed on a plaster and each resulting slurry was poured thereinto, impressed for 15 minutes impress, and air-dried overnight. Thus, disk-shaped compacts were obtained. Each disk-shaped compact was observed using a scanning electron microscope (S-4300 manufactured by Hitachi, Ltd.) to prepare a thickness distribution. As a result of SEM observation, it was observed that a thickness decreased in the case of the delaminated compound that had been refluxed for 96 hours in Example 5 (Fig. 7), compared with the mica powder before delamination treatment (Fig. 6). Fig. 8 is a graph indicating a thickness distribution of the particular complex after delamination treatment obtained in Example 5 (delaminated compound) and that of the mica powder before delamination treatment obtained in Example 1. It was observed in the thickness distribution that delamination proceeded in the order of the mica powder before delamination treatment ((a) in Fig. 8; $T_{50}$: 109 nm), the delaminated compound of Example 1 ((b) in Fig. 8; $T_{50}$: 52 nm), and the delaminated compounds that had been refluxed 48 hours ((c) in Fig. 8; $T_{50}$: 41 nm), 72 hours ((d) in Fig. 8; $T_{50}$: 36 nm), and 96 hours ((e) in Fig. 8; $T_{50}$: 32 nm) of Example 5. It was shown that as the intercalation quantity increases, delamination proceeds.

Further, as a result of measurement of average particle diameters of the delaminated compounds of Examples 1 and 5 by a laser diffraction scattering particle diameter distribution analyzer, the average particle diameters were 3.57 $\mu$m (Example 1), 4.00 $\mu$m (Example 5, 48 hours), 4.26 $\mu$m (Example 5, 72 hours), and 4.35 $\mu$m (Example 5, 96 hours).

[Example 7]

(Synthesis of Catechol Resorcinol Novolac (CRN) Resin)

627 g of resorcinol, 33 g of catechol, 316.2 g of 37% by mass formalin, 15 g of oxalic acid, and 300 g of water were introduced into a 3-L separable flask equipped with a stirrer, a cooler, and a thermometer. The temperature was increased to 100°C with heating using an oil bath, and a reaction was made to proceed at the reflux temperature for 4 hours. Then, the temperature inside of the flask was increased to 170°C while water was distilled away. The reaction was made to proceed for 8 hours while the temperature was maintained at 170°C.

Thereafter, condensation was carried out under reduced pressure for 20 minutes to eliminate water and the like in the system. Then, a catechol resorcinol novolac resin was taken out. A number average molecular weight and a weight average molecular weight of the obtained catechol resorcinol novolac resin were 530 and 930, respectively. In addition, an equivalent amount of a hydroxyl group in the catechol resorcinol novolac resin was 65g/eq. The catechol resorcinol novolac resin obtained from the above process was used in the Examples described below.

To a 100-cm$^3$ polyethylene bottle, 0.0960 parts by mass of N-phenyl-3-aminopropyltrimethoxysilane (manufactured by Shin-Etsu Chemical Co., Ltd.; product name: "KBM-573") serving as a coupling agent and 4.6680 parts by mass of the cyclohexanone dissolution product of the catechol resorcinol novolac resin synthesized above (solid content:

50% by mass) serving as a curing agent were added in this order.

Subsequently, 120.00 parts by mass of alumina balls (particle diameter: 3 mm) were introduced into the polyethylene bottle. Then, as inorganic fillers, 59.51 parts by mass of aluminum oxide having an average particle diameter of 18 $\mu$m (AA-18, manufactured by Sumitomo Chemical Co., Ltd.), 21.64 parts by mass of aluminum oxide having an average particle diameter of 3 $\mu$m (AA-3, manufactured by Sumitomo Chemical Co., Ltd.), and 9.02 parts by mass of aluminum oxide having an average particle diameter of 0.4 $\mu$m (AA-04, manufactured by Sumitomo Chemical Co., Ltd.) were added. Thereafter, 0.4248 parts by mass of the delaminated compound obtained in Example 5 (96 hours) (c axis direction thickness: 32 nm; average particle diameter: 4.35 $\mu$m) was added.

Further, 14.33 parts by mass of methylethylketone and 2.44 parts by mass of cyclohexanone were added and mixed. After mixing, 7.2170 parts by mass of 1-{(3-methyl-4-oxiranylmethoxy)phenyl}-4-(4-oxiranylmethoxyphenyl)-1-cyclohexene synthesized from 1-(3-methyl-4-hydroxyphenyl)-4-(4-hydroxyphenyl)-1-cyclohexene and epichlorohydrin (epoxy resin) and 0.0760 parts by mass of triphenylphosphine (curing catalyst manufactured by Wako Pure Chemical Industries, Ltd.) were added and further mixed, followed by ball mill crushing for from 40 hours to 60 hours. Thus, resin sheet coating liquid was obtained as an insulating resin composition.

The obtained resin sheet coating liquid was applied to a mold-releasing face of a polyethyleneterephthalate film (manufactured by Fujimori Kogyo Co., Ltd., 75E-0010CTR-4, herienafter abbreviated as a "PET film") using an applicator so that the thickness becomes approximately 300 $\mu$m. The resulting coating was left under ordinary conditions for 15 minutes, followed by drying in a box-type oven at 100°C for 30 minutes. Thus, a resin composition layer was formed on the PET film. Subsequently, an upper face of the resin composition layer, which had been exposed to the air, was covered with another PET film and heat-pressed (upper heating plate: 150°C; lower heating plate: 80°C; pressure: 1.5 MPa; treatment time: 3 minutes) for performing a flattening treatment. Thus, a B stage sheet was obtained as a resin sheet having a thickness of 200 $\mu$m.

The PET films were removed from both sides of the resin sheet (B stage sheet) obtained by the above method, the resin sheet was sandwiched on both sides by a copper foil having a thickness of 105 $\mu$m (manufactured by Furukawa Electric Co., Ltd., GTS FOIL), subjected to vacuum heat press (upper heating plate: 150°C; lower heating plate: 80°C; degree of vacuum: not more than 1 kPa; pressure: 4 MPa; treatment time: 7 minutes), and placed in a box-type oven for curing by stepped curing at 140°C for 2 hours, 165°C for 2 hours, and 190°C for 2 hours. Copper was eliminated from the obtained cured product sandwiched by the copper foil by etching using a sodium persulfate solution. Thus, a cured product of the insulating resin sheet was obtained.

Thermal conductivity of the obtained cured product was measured by the xenon flash method as described below. As a result, the thermal conductivity was found as 8.3 W/(m·K).

In addition, as a result of measurement of insulation by the BDV (Break Down Voltage) method as described below, the lowest value was 25.1 kV/mm, and the mean value was 25.9 kV/mm.

(Method of Measuring Thermal conductivity)

A NANOFLASH LFA447 type thermal diffusivity analyzer for a Xe flash method manufactured by NETZSCH was used for measurement of thermal diffusivity of the sheet. A thermal conductivity (W/(m·K)) was calculated by multiplying the numerical value of the obtained thermal diffusivity by specific heat Cp (J/g·K) and density d (g/cm$^3$). All measurements were carried out at 25 ± 1°C.

(Insulating Property)

The resin sheet cured product obtained as described above was hold with cylindrical electrodes having a diameter of 25 mm and subjected to measurement at a pressure increase speed of 500V/s, an alternating current of 50 Hz, a step voltage of 0.50 kV, a voltage retention time of 60 s, and 25°C in oil using an dielectric breakdown tester DAC-6032C manufactured by Soken Electric Co., Ltd.

[Example 8]

To a 100-cm$^3$ polyethylene bottle, 0.0960 parts by mass of N-phenyl-3-aminopropyltrimethoxysilane (manufactured by Shin-Etsu Chemical Co., Ltd.; product name: "KBM-573") serving as a coupling agent and 4.6680 parts by mass of the cyclohexanone dissolution product of the catechol resorcinol novolac resin synthesized above (solid content 50% by mass) serving as a curing agent were added in that order.

Subsequently, 120.00 parts by mass of alumina balls (particle diameter: 3 mm) were introduced into the above polyethylene bottle. Then, as inorganic fillers, 59.51 parts by mass of aluminum oxide having an average particle diameter of 18 $\mu$m (AA-18, manufactured by Sumitomo Chemical Co., Ltd.), 21.64 parts by mass of aluminum oxide having an average particle diameter of 3 $\mu$m (AA-3, manufactured by Sumitomo Chemical Co., Ltd.), and 9.02 parts by mass of aluminum oxide having an average particle diameter of 0.4 μm (AA-04, manufactured by Sumitomo Chemical Co., Ltd.) were added. Thereafter, 0.8496 parts by mass of the delaminated compound obtained in Example 5 (96 hours) (c axis direction thickness: 32 nm; average particle diameter: 4.35 μm) was added.

Further, 15.04 parts by mass of methylethylketone and 2.68 parts by mass of cyclohexanone were added and mixed. After mixing, 7.2170 parts by mass of 1-{(3-methyl-4-oxiranylmethoxy)phenyl}-4-(4-oxiranylmethoxyphenyl)-1-cyclohexene synthesized from 1-(3-methyl-4-hydroxyphenyl)-4-(4-hydroxyphenyl)-1-cyclohexene and epichlorohydrin (epoxy resin) and 0.0760 parts by mass of triphenylphosphine (curing catalyst manufactured by Wako Pure Chemical Industries, Ltd.) were added and further mixed, followed by ball mill crushing for from 40 hours to 60 hours. Thus, resin sheet coating liquid was obtained as an insulating resin composition.

The obtained resin sheet coating liquid was applied to a mold-releasing face of a PET film using an applicator so that the thickness became approximately 300 μm. The resulting coating was left under ordinary conditions for 15 minutes and dried in a box-type oven at 100°C for 30 minutes. Thus, a resin composition layer was formed on the PET film. Subsequently, an upper face of the resin composition layer, which had been exposed to the air, was covered by another PET film and heat-pressed (upper heating plate: 150°C; lower heating plate: 80°C; pressure: 1.5 MPa; treatment time: 3 minutes) for performing a flattening treatment. Thus, a B stage sheet was obtained as a resin sheet having a thickness of 200 μm.

The PET films were removed from both sides of the resin sheet (B stage sheet) obtained by the above method, the resin sheet was sandwiched on both sides by a copper foil having a thickness of 105 μm (manufactured by Furukawa Electric Co., Ltd., GTS FOIL), subjected to vacuum heat press (upper heating plate: 150°C; lower heating plate: 80°C; degree of vacuum: not more than 1 kPa; pressure: 4 MPa; treatment time: 7 minutes), and placed in a box-type oven for curing by stepped curing at 140°C for 2 hours, 165°C for 2 hours, and 190°C for 2 hours. Copper was eliminated from the obtained cured product sandwiched by the copper foil by etching using a sodium persulfate solution. Thus, a cured product of the insulating resin sheet was obtained.

As a result of measurement of thermal conductivity of the obtained cured product by the xenon flash method, the thermal conductivity was found as 8.0 W/(m·K).

In addition, as a result of measurement of insulation by the BDV method, the lowest value was 25.6 kV/mm and the mean value was 28.4 kV/mm.

[Example 9]

To a 250-cm$^3$ polyethylene bottle, 4.1190 parts by mass of a cyclohexanone dissolution product of the catechol resorcinol novolac resin synthesized as above (solid content 50% by mass) serving as a curing agent, 6.6775 parts by mass of 1-{(3-methyl-4-oxiranylmethoxy)phenyl}-4-(4-oxiranylmethoxyphenyl)-1-cyclohexene synthesized from 1-(3-methyl-4-hydroxyphenyl)-4-(4-hydroxyphenyl)-1-cyclohexene and epichlorohydrin (epoxy resin), 0.0707 parts by mass of triphenylphosphine (curing catalyst manufactured by Wako Pure Chemical Industries, Ltd.), and 25.90 parts by mass of cyclohexanone (manufactured by Wako Pure Chemical Industries, Ltd.) were added and mixed. Then, 37.38 parts by mass of boron nitride particles (volume average particle diameter: 40 μm; manufactured by Mizushima Ferroalloy Co., Ltd.; product name: "HP-40MF100") serving as an inorganic filler and 0.3188 parts by mass of the delaminated compound (c axis direction thickness: 32 nm; average particle diameter: 4.35 μm) obtained in Example 5 (96 hours) were added and further mixed. Thus, resin sheet coating liquid was obtained as an insulating resin composition.

The obtained resin sheet coating liquid was applied to a mold-releasing face of a PET film using an applicator so that the thickness became approximately 300 μm. The resulting coating was left under ordinary conditions for 10 minutes and then dried in a box-type oven at 100°C for 10 minutes to form a resin composition layer on the PET film. Thus, a resin composition layer was formed on the PET film. Two sheets of the PET film on which the resin composition layer was formed were stacked in such a manner that the resin composition layers faced with each other and the sheets were heat-pressed (upper heating plate: 150°C; lower heating plate: 150°C; pressure: 15 MPa; treatment time: 4 minutes) for performing a flattening treatment. Thus, a B stage sheet was obtained as a resin sheet having a thickness of 200 μm.

The PET films were removed from both sides of the resin sheet (B stage sheet) obtained by the above method, the resin sheet was sandwiched on both sides by a copper foil having a thickness of 105 μm (manufactured by Furukawa Electric Co., Ltd., GTS FOIL), subjected to vacuum heat press (upper heating plate: 170°C; lower heating plate: 170°C; degree of vacuum: not more than 1 kPa; pressure: 10 MPa; treatment time: 7 minutes), and placed in a box-type oven for curing in curing steps of 160°C for 30 minutes and 190°C for 2 hours. Copper was eliminated from the obtained cured product sandwiched by the copper foil by etching using a sodium persulfate solution. Thus, a cured product of the insulating resin sheet was obtained.

As a result of measurement of thermal conductivity of the obtained cured product by the xenon flash method, the thermal conductivity was found as 10.0W/(m·K).

In addition, as a result of measurement of insulation by the BDV method, the lowest value was 28.5kV/mm and the mean value was 30.4kV/mm.

[Example 10]

To a 100-cm$^3$ polyethylene bottle, 0.0960 parts by mass of N-phenyl-3-aminopropyltrimethoxysilane (manufactured by Shin-Etsu Chemical Co., Ltd.; product name "KBM-573") serving as a coupling agent and 4.6680 parts by mass of the cyclohexanone dissolution product of the catechol resorcinol novolac resin synthesized above (solid content 50% by mass) serving as a curing agent were added in that order.

Subsequently, 120.00 parts by mass of alumina balls (particle diameter: 3 mm) were introduced into the above polyethylene bottle. Then, 49.8427 parts by mass of silicone dioxide having an average particle diameter of 4.0 $\mu$m (E-03, manufactured by Tokai Minerals) was added as an inorganic filler. Thereafter, 0.4248 parts by mass of the delaminated compound (c axis direction thickness: 32 nm; average particle diameter: 4.35 $\mu$m) obtained in Example 5 (96 hours) was added.

Further, 17.19 parts by mass of methylethylketone and 3.40 parts by mass of cyclohexanone were added and mixed. After mixing, 7.2170 parts by mass of 1-{(3-methyl-4-oxiranylmethoxy)phenyl}-4-(4-oxiranylmethoxyphenyl)-1-cyclohexene synthesized from 1-(3-methyl-4-hydroxyphenyl)-4-(4-hydroxyphenyl)-1-cyclohexene and epichlorohydrin (epoxy resin) and 0.0760 parts by mass of triphenylphosphine (curing catalyst manufactured by Wako Pure Chemical Industries, Ltd.) were added and further mixed, followed by ball mill crushing for from 40 hours to 60 hours. Thus, resin sheet coating liquid was obtained as an insulating resin composition.

The obtained resin sheet coating liquid was applied to a mold-releasing face of a PET film using an applicator so that the thickness became approximately 300 $\mu$m. The resulting coating was left under ordinary conditions for 15 minutes and dried in a box-type oven at 100°C for 30 minutes. Thus, a resin composition layer was formed on the PET film. Subsequently, an upper face of the resin composition layer, which had been exposed to the air, was covered by another PET film and heat-pressed (upper heating plate: 150°C; lower heating plate: 80°C; pressure: 1.5 MPa; treatment time: 3 minutes) for performing a flattening treatment. Thus, a B stage sheet was obtained as a resin sheet having a thickness of 200 $\mu$m.

The PET films were removed from both sides of the resin sheet (B stage sheet) obtained by the above method, the resin sheet was sandwiched on both sides by a copper foil having a thickness of 105 $\mu$m (manufactured by Furukawa Electric Co., Ltd., GTS FOIL), subjected to vacuum heat press (upper heating plate: 150°C; lower heating plate: 80°C; degree of vacuum: not more than 1 kPa; pressure: 4 MPa; treatment time: 7 minutes), and placed in a box-type oven for curing by stepped curing at 140°C for 2 hours, 165°C for 2 hours, and 190°C for 2 hours. Copper was eliminated from the obtained cured product sandwiched by the copper foil by etching using a sodium persulfate solution. Thus, a cured product of the insulating resin sheet was obtained.

As a result of measurement of thermal conductivity of the obtained cured product by the xenon flash method, the thermal conductivity was found as 1.4W/(m·K).

In addition, as a result of measurement of insulation by the BDV method, the lowest value was 18.5kV/mm and the mean value was 20.5kV/mm.

[Comparative Example 3]

To a 100-cm$^3$ polyethylene bottle, 0.0960 parts by mass of N-phenyl-3-aminopropyltrimethoxysilane (manufactured by Shin-Etsu Chemical Co., Ltd.; product name "KBM-573") serving as a coupling agent and 4.6680 parts by mass of the cyclohexanone dissolution product of the catechol resorcinol novolac resin synthesized above (solid content 50% by mass) serving as a curing agent were added in that order.

Subsequently, 120.00 parts by mass of alumina balls (particle diameter: 3 mm) were introduced into the above polyethylene bottle. Then, 59.51 parts by mass of aluminum oxide having an average particle diameter of 18 $\mu$m (AA-18) (manufactured by Sumitomo Chemical Co., Ltd.) serving as an inorganic filler, 21.64 parts by mass of aluminum oxide having an average particle diameter of 3 $\mu$m (AA-3) (manufactured by Sumitomo Chemical Co., Ltd.), and 9.02 parts by mass of aluminum oxide having an average particle diameter of 0.4 $\mu$m (AA-04) (manufactured by Sumitomo Chemical Co., Ltd.) were added.

Further, 14.33 parts by mass of methylethylketone and 2.44 parts by mass of cyclohexanone were added and mixed. After mixing, 7.2170 parts by mass of 1-{(3-methyl-4-oxiranylmethoxy)phenyl}-4-(4-oxiranylmethoxyphenyl)-1-cyclohexene that had been synthesized from 1-(3-methyl-4-hydroxyphenyl)-4-(4-hydroxyphenyl)-1-cyclohexene and epichlorohydrin (epoxy resin) and 0.0760 parts by mass of triphenylphosphine (curing catalyst manufactured by Wako Pure Chemical Industries, Ltd.) were added and further mixed, followed by ball mill crushing for 40 hours to 60 hours. Thus, resin sheet coating liquid was obtained as an insulating resin composition.

The obtained resin sheet coating liquid was applied to a mold-releasing face of a PET film using an applicator so that the thickness became approximately 300 $\mu$m. The resulting coating was left under ordinary conditions for 15 minutes and dried in a box-type oven at 100°C for 30 minutes to form a resin composition layer on the PET film. Thus, a resin composition layer was formed on the PET film. Subsequently, an upper face of the resin composition layer, which had been exposed to the air, was covered by another PET film and heat-pressed (upper heating plate: 150°C; lower heating plate: 80°C; pressure: 1.5 MPa; treatment time: 3 minutes) for performing a flattening treatment. Thus, a B stage sheet was obtained as a resin sheet having a thickness of 200 $\mu$m.

The PET films were removed from both sides of the resin sheet (B stage sheet) obtained by the above-mentioned method, the resin sheet was sandwiched on both sides by a copper foil having a thickness of 105 $\mu$m (manufactured by Furukawa Electric Co., Ltd., GTS FOIL), subjected to vacuum heat press (upper heating plate: 150°C; lower heating plate: 80°C; degree of vacuum: not more than 1 kPa; pressure: 4 MPa; treatment time: 7 minutes), and placed in a box-type oven for curing by stepped curing at 140°C for 2 hours, 165°C for 2 hours, and 190°C for 2 hours. Copper was eliminated from the obtained cured product sandwiched by the copper foil by etching using a sodium persulfate solution. Thus, a cured product of the insulating resin sheet was obtained.

As a result of measurement of thermal conductivity of the obtained cured product by the xenon flash method, the thermal conductivity was found as 8.9W/(m·K).

In addition, as a result of measurement of insulation by the BDV method, the lowest value was 19.5kV/mm and the mean value was 25.4kV/mm.

[Comparative Example 4]

To a 250-cm$^3$ polyethylene bottle, 4.1190 parts by mass of a cyclohexanone dissolution product of the catechol resorcinol novolac resin synthesized as above (solid content: 50% by mass) serving as a curing agent, 6.6775 parts by mass of 1-{(3-methyl-4-oxiranylmethoxy)phenyl}-4-(4-oxiranylmethoxyphenyl)-1-cyclohexene synthesized from 1-(3-methyl-4-hydroxyphenyl)-4-(4-hydroxyphenyl)-1-cyclohexene and epichlorohydrin (epoxy resin), 0.0707 parts by mass of triphenylphosphine (curing catalyst manufactured by Wako Pure Chemical Industries, Ltd.), and 25.90 parts by mass of cyclohexanone (manufactured by Wako Pure Chemical Industries, Ltd.) were added and mixed. Then, 37.38 parts by mass of boron nitride particles (volume average particle diameter: 40 $\mu$m; manufactured by Mizushima Ferroalloy Co., Ltd.; product name: "HP-40MF100") were add as an inorganic filler and further mixed. Thus, resin sheet coating liquid was obtained as an insulating resin composition.

The obtained resin sheet coating liquid was applied to a mold-releasing face of a PET film using an applicator so that the thickness became approximately 300 $\mu$m. The resulting coating was left under ordinary conditions for 10 minutes and then dried in a box-type oven at 100°C for 10 minutes. Thus, a resin composition layer was formed on the PET film. Two sheets of the PET film on which the resin composition layer was formed were stacked in such a manner that the resin composition layers faced with each other and the sheets were heat-pressed (upper heating plate: 150°C; lower heating plate: 150°C; pressure: 15 MPa; treatment time: 4 minutes) for performing a flattening treatment. Thus, a B stage sheet was obtained as a resin sheet having a thickness of 200 $\mu$m.

The PET films were removed from both sides of the resin sheet (B stage sheet) obtained by the above method, the resin sheet was sandwiched on both sides by a copper foil having a thickness of 105 $\mu$m (manufactured by Furukawa Electric Co., Ltd., GTS FOIL), subjected to vacuum heat press (upper heating plate: 170°C; lower heating plate: 170°C; degree of vacuum: not more than 1 kPa; pressure: 10 MPa; treatment time: 7 minutes), and placed in a box-type oven for curing in curing steps of 160°C for 30 minutes and 190°C for 2 hours. Copper was eliminated from the obtained cured product sandwiched by the copper foil by etching using a sodium persulfate solution. Thus, a cured product of the insulating resin sheet was obtained.

As a result of measurement of thermal conductivity of the obtained cured product by the xenon flash method, the thermal conductivity was found as 10.1W/(m·K).

In addition, as a result of measurement of insulation by the BDV method, the lowest value was 25.6 kV/mm and the mean value was 30.0kV/mm.

[Comparative Example 5]

To a 100-cm$^3$ polyethylene bottle, 0.0960 parts by mass of N-phenyl-3-aminopropyltrimethoxysilane (manufactured by Shin-Etsu Chemical Co., Ltd.; product name: "KBM-573") serving as a coupling agent and 4.6680 parts by mass of the cyclohexanone dissolution product of the catechol resorcinol novolac resin synthesized above (solid content 50% by mass) as a curing agent were added in that order.

Subsequently, 120.00 parts by mass of alumina balls (particle diameter: 3 mm) were introduced into the above-mentioned polyethylene bottle, and 49.8427 parts by mass of silicone dioxide having an average particle diameter of 4.0 $\mu$m (E-03) (manufactured by Tokai Minerals) was added as an inorganic filler.

Further, 17.19 parts by mass of methylethylketone and 3.40 parts by mass of cyclohexanone were added and mixed. After mixing, 7.2170 parts by mass of 1-{(3-methyl-4-oxiranylmethoxy)phenyl}-4-(4-oxiranylmethoxyphenyl)-1-cyclohexene synthesized from 1-(3-methyl-4-hydroxyphenyl)-4-(4-hydroxyphenyl)-1-cyclohexene and epichlorohydrin (epoxy resin) and 0.0760 parts by mass of triphenylphosphine (curing catalyst manufactured by Wako Pure Chemical Industries, Ltd.) were added and further mixed, followed by ball mill crushing for 40 hours to 60 hours. Thus, resin sheet coating liquid was obtained as an insulating resin composition.

The obtained resin sheet coating liquid was applied to a mold-releasing face of a PET film using an applicator so that the thickness became approximately 300 $\mu$m. The resulting coating was left under ordinary conditions for 15 minutes and dried in a box-type oven at 100°C for 30 minutes. Thus, a resin composition layer was formed on the PET film. Subsequently, an upper face of the resin composition layer, which had been exposed to the air, was covered by another PET film and heat-pressed (upper heating plate: 150°C; lower heating plate: 80°C; pressure: 1.5 MPa; treatment time: 3 minutes) for performing a flattening treatment. Thus, a B stage sheet was obtained as a resin sheet having a thickness of 200 $\mu$m.

The PET films were removed from both sides of the resin sheet (B stage sheet) obtained by the above method, the resin sheet was sandwiched on both sides by a copper foil having a thickness of 105 $\mu$m (manufactured by Furukawa Electric Co., Ltd., GTS FOIL), subjected to vacuum heat press (upper heating plate: 150°C; lower heating plate: 80°C; degree of vacuum: not more than 1 kPa; pressure: 4 MPa; treatment time: 7 minutes), and placed in a box-type oven for curing by stepped curing at 140°C for 2 hours, 165°C for 2 hours, and 190°C for 2 hours. Copper was eliminated from the obtained cured product sandwiched by the copper foil by etching using a sodium persulfate solution. Thus, a cured product of the insulating resin sheet was obtained.

As a result of measurement of thermal conductivity of the obtained cured product by the xenon flash method, the thermal conductivity was found as 1.5W/(m·K).

In addition, as a result of measurement of insulation by the BDV method, the lowest value was 15.1kV/mm and the mean value was 20.0kV/mm.

Table 2 summarizes the results of examination of thermal conductivity and BDV for the thermally-conductive insulating sheets prepared in Examples 7 to 10 and Comparative Examples 3 to 5

[Table 2]

| | Resin | Filler | | Delaminated compound content (% by volume) | | Thermal conductivity (W/(m·K)) | Dielectric breakdown electric field (kV/mm) | |
|---|---|---|---|---|---|---|---|---|
| | | Type | Content (% by volume) | Percentage with respect to filler | Content in sheet | | Minimum | Average |
| Example 7 | Epoxy-phenol curing system | Aluminum oxide | 74 | 0.75 | 0.55 | 8.3 | 25.1 | 25.9 |
| Example 8 | Epoxy-phenol curing system | Aluminum oxide | 74 | 1.5 | 1.10 | 8.0 | 25.6 | 28.4 |
| Example 9 | Epoxy-phenol curing system | Boron nitride | 70 | 0.75 | 0.53 | 10.0 | 28.5 | 30.4 |
| Example 10 | Epoxy-phenol curing system | Silicon dioxide | 74 | 0.75 | 0.55 | 1.4 | 18.5 | 20.5 |
| Comparative Example 3 | Epoxy-phenol curing system | Aluminum oxide | 74 | 0 | 0 | 8.9 | 19.5 | 25.4 |
| Comparative Example 4 | Epoxy-phenol curing system | Boron nitride | 70 | 0 | 0 | 10.1 | 25.6 | 30.0 |
| Comparative Example 5 | Epoxy-phenol curing system | Silicon dioxide | 74 | 0 | 0 | 1.5 | 15.1 | 20.0 |

As is understood from Table 2, the resin sheets composed of the insulating resin composition of the invention are excellent in terms of insulation because of the addition of the delaminated compound of the invention.

Japanese Patent Application No. 2015-43961, filed March 5, 2015, is hereby incorporated by reference in its entirety.

All references, patent applications, and technical standards described herein are incorporated by reference to the same extent as if each of the publications, patent applications, and technical standards has been written specifically and individually to be incorporated by reference.

**Claims**

1.  A method of producing a complex of a lamellar inorganic compound and an organic compound, the method comprising:

    heat-treating a non-swelling lamellar inorganic compound within a pyrolysis temperature range of the non-swelling lamellar inorganic compound; and
    intercalating an organic compound into the non-swelling lamellar inorganic compound in a dispersion liquid in which the heat-treated non-swelling lamellar inorganic compound is dispersed in a medium, thereby inserting the organic compound into an interlamellar space of the non-swelling lamellar inorganic compound,

    wherein the non-swelling lamellar inorganic compound comprises unit crystal layers disposed one on another to form a lamellar structure,
    the non-swelling lamellar inorganic compound would expand in its c axis direction by from 0.05Å to 0.20Å when the non-swelling lamellar inorganic compound is heated at a pyrolysis upper limit temperature of the non-swelling lamellar inorganic compound for 1 hour, and
    a crystal structure of the unit crystal layers would not change when the non-swelling lamellar inorganic compound is heated at the pyrolysis upper limit temperature for 1 hour.

2.  The method of producing a complex of a lamellar inorganic compound and an organic compound according to claim 1, wherein the non-swelling lamellar inorganic compound is mica.

3.  The method of producing a complex of a lamellar inorganic compound and an organic compound according to claim 1 or 2, wherein the organic compound is at least one cationic organic compound selected from the group consisting of an amine salt, a phosphonium salt, an imidazolium salt, a pyridinium salt, a sulfonium salt, and an iodonium salt.

4.  The method of producing a complex of a lamellar inorganic compound and an organic compound according to any one of claims 1 to 3, wherein a concentration of the organic compound in the dispersion liquid is 0.01 mol/L or more but not more than a solubility of the organic compound, and wherein a content of the non-swelling lamellar inorganic compound in the dispersion liquid is from 0.5% by volume to 50% by volume.

5.  A method of producing a delaminated lamellar inorganic compound, the method comprising:

    heat-treating a non-swelling lamellar inorganic compound within a pyrolysis temperature range of the non-swelling lamellar inorganic compound;
    intercalating an organic compound into the non-swelling lamellar inorganic compound in a dispersion liquid in which the heat-treated non-swelling lamellar inorganic compound is dispersed in a medium, thereby inserting the organic compound into an interlamellar space of the non-swelling lamellar inorganic compound; and
    applying a shear force to the dispersion liquid via a mechanical treatment, thereby delaminating the non-swelling lamellar inorganic compound comprising the intercalation,

    wherein the non-swelling lamellar inorganic compound comprises unit crystal layers disposed one on another to form a lamellar structure,
    the non-swelling lamellar inorganic compound would expand in its c axis direction by from 0.05Å to 0.20Å when the non-swelling lamellar inorganic compound is heated at a pyrolysis upper limit temperature of the non-swelling lamellar inorganic compound for 1 hour, and
    a crystal structure of the unit crystal layers would not change when the non-swelling lamellar inorganic compound is heated at the pyrolysis upper limit temperature for 1 hour.

**6.** The method of producing a delaminated lamellar inorganic compound according to claim 5, wherein an equilibrium filler density of the dispersion liquid after the application of the shear force to the dispersion liquid is not more than 30% by volume.

**7.** The method of producing a delaminated lamellar inorganic compound according to claim 5 or 6, wherein an average particle diameter of the delaminated non-swelling lamellar inorganic compound after the application of the shear force to the dispersion liquid is from 50% to 100% of an average particle diameter of the non-swelling lamellar inorganic compound comprising the intercalation before the application of the shear force to the dispersion liquid.

**8.** The method of producing a delaminated lamellar inorganic compound according to any one of claims 5 to 7, wherein an impingement pressure of the dispersion liquid employed in the mechanical treatment is from 50 MPa to 250 MPa.

**9.** A complex of a lamellar inorganic compound and an organic compound,
the complex comprising the organic compound intercalated into a non-swelling lamellar inorganic compound,
the non-swelling lamellar inorganic compound comprising unit crystal layers disposed one on another to form a lamellar structure,
the non-swelling lamellar inorganic compound expanding in its c axis direction by from 0.05Å to 0.20Å when the non-swelling lamellar inorganic compound is heated at an upper limit of a pyrolysis temperature thereof for 1 hour, and a crystal structure of the unit crystal layers would not change when the non-swelling lamellar inorganic compound is heated at the pyrolysis upper limit temperature for 1 hour.

**10.** The complex of a lamellar inorganic compound and an organic compound according to claim 9, wherein the organic compound that is intercalated into an interlamellar space of the non-swelling lamellar inorganic compound accounts for from 1% by mass to 40% by mass with respect to 100% by mass of the non-swelling lamellar inorganic compound.

**11.** A delaminated lamellar inorganic compound, having an average particle thickness of from 1 nm to 80 nm in its c axis direction.

**12.** The delaminated lamellar inorganic compound according to claim 11, having an average particle diameter that is from 50% to 100% of an average particle diameter of a non-swelling lamellar inorganic compound comprising intercalation.

**13.** An insulating resin composition, comprising a thermosetting resin and an inorganic filler, at least a part of the inorganic filler being the delaminated lamellar inorganic compound according to claim 11 or 12.

**14.** The insulating resin composition according to claim 13, wherein the delaminated lamellar inorganic compound accounts for from 0.5% by volume to 10% by volume of the inorganic filler.

**15.** A resin sheet obtained by forming the insulating resin composition according to claim 13 or 14 into a sheet shape.

**16.** An insulator that is a cured product of the insulating resin composition according to claim 13 or 14.

**17.** A resin sheet cured product that is a heat-treated product of the resin sheet according to claim 15.

**18.** A heat dissipating member, comprising: a metal work; and the resin sheet according to claim 15 or the resin sheet cured product according to claim 17 disposed on the metal work.

FIG. 1

FIG. 2

FIG. 3

FIG. 4

FIG. 5

FIG. 6

FIG. 7

FIG. 8

## INTERNATIONAL SEARCH REPORT

| | International application No. |
|---|---|
| | PCT/JP2016/056670 |

**A. CLASSIFICATION OF SUBJECT MATTER**
*C01B33/44*(2006.01)i, *C01B33/42*(2006.01)i, *C08K9/04*(2006.01)i, *C08L101/00*(2006.01)i

According to International Patent Classification (IPC) or to both national classification and IPC

**B. FIELDS SEARCHED**

Minimum documentation searched (classification system followed by classification symbols)
C01B33/44, C01B33/42, C08K9/04, C08L101/00

Documentation searched other than minimum documentation to the extent that such documents are included in the fields searched
Jitsuyo Shinan Koho        1922–1996    Jitsuyo Shinan Toroku Koho    1996–2016
Kokai Jitsuyo Shinan Koho   1971–2016    Toroku Jitsuyo Shinan Koho    1994–2016

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br><br>A | WO 2007/046305 A1 (Independent Administrative Institution National Institute for Materials Science),<br>26 April 2007 (26.04.2007),<br>claims 1 to 12; paragraphs [0034], [0039], [0040], [0044], [0051], [0052], [0056] to [0062], [0070] to [0078]; fig. 1 to 4<br>& US 2009/0227715 A1<br>claims 1 to 12; paragraphs [0020], [0022], [0027], [0028], [0032], [0036], [0039], [0040], [0044] to [0048], [0059] to [0070]; fig. 1 to 4 | 1-3,5,9,<br>11-13,15-18<br>4,6-8,10,14 |

| ☒ | Further documents are listed in the continuation of Box C. | ☐ | See patent family annex. |
|---|---|---|---|

| * | Special categories of cited documents: |
|---|---|
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>20 April 2016 (20.04.16) | Date of mailing of the international search report<br>10 May 2016 (10.05.16) |
|---|---|
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

International application No.

PCT/JP2016/056670

C (Continuation).　DOCUMENTS CONSIDERED TO BE RELEVANT

| Category* | Citation of document, with indication, where appropriate, of the relevant passages | Relevant to claim No. |
|---|---|---|
| A | JP 11-060983 A　(Amcol International Corp.), 05 March 1999 (05.03.1999), claims 1 to 40; paragraphs [0185] to [0207] & US 6057396 A claims 1 to 47; column 29, line 44 to column 32, line 14 & EP 846661 A2　　　　& CA 2218303 A1 | 1-18 |
| A | JP 2008-063408 A　(Independent Administrative Institution National Institute for Materials Science), 21 March 2008 (21.03.2008), entire text (Family: none) | 1-18 |
| A | JP 2000-053805 A　(Showa Denko Kabushiki Kaisha), 22 February 2000 (22.02.2000), entire text (Family: none) | 1-18 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008075069 A
- JP H9208745 A
- JP 2008007753 A
- JP H987096 A
- JP S63215775 A
- JP 2004169030 A

- JP 2009191239 A
- JP 2012158622 A
- JP 2008063408 A
- WO 200622431 A
- JP 2005206814 A
- JP 2015043961 A